(19) Europäisches Patentamt
European Patent Office
Office européen des brevets

(11) EP 2 172 456 A1

(12) EUROPEAN PATENT APPLICATION
published in accordance with Art. 153(4) EPC

(43) Date of publication:
**07.04.2010 Bulletin 2010/14**

(21) Application number: **08765779.7**

(22) Date of filing: **20.06.2008**

(51) Int Cl.:
***C07D 213/38*** (2006.01) ***A01N 43/40*** (2006.01)
***A01N 43/60*** (2006.01) ***A01N 43/80*** (2006.01)
***A01P 7/02*** (2006.01) ***A01P 7/04*** (2006.01)
***C07D 213/61*** (2006.01) ***C07D 213/64*** (2006.01)
***C07D 213/70*** (2006.01) ***C07D 213/89*** (2006.01)
***C07D 401/12*** (2006.01) ***C07D 401/14*** (2006.01)
***C07D 407/12*** (2006.01) ***C07D 409/12*** (2006.01)
***C07D 413/12*** (2006.01) ***C07D 417/12*** (2006.01)

(86) International application number:
**PCT/JP2008/061359**

(87) International publication number:
**WO 2009/001784 (31.12.2008 Gazette 2009/01)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **22.06.2007 JP 2007165618**

(71) Applicant: **ISHIHARA SANGYO KAISHA, LTD.**
**Osaka-shi**
**Osaka 550-0002 (JP)**

(72) Inventors:
- **MORITA, Masayuki**
  **Kusatsu-shi**
  **Shiga 525-0025 (JP)**
- **YAMAMOTO, Kazuhiro**
  **Kusatsu-shi**
  **Shiga 525-0025 (JP)**
- **UEKI, Toshihiko**
  **Kusatsu-shi**
  **Shiga 525-0025 (JP)**
- **AZUMA,Kumiko**
  **Kusatsu-shi**
  **Shiga 525-0025 (JP)**
- **HAMAMOTO, Taku**
  **Kusatsu-shi**
  **Shiga 525-0025 (JP)**

(74) Representative: **Hartz, Nikolai et al**
**Wächtershäuser & Hartz**
**Patentanwälte**
**Weinstrasse 8**
**80333 München (DE)**

# (54) N-PHENYL-METHANAMINE DERIVATIVE AND PEST CONTROL AGENT CONTAINING THE SAME

(57) A novel pesticide is provided. The present invention provides a pesticide containing, as an active ingredient, an N-phenyl-methanamine derivative represented by the formula (I) or its salt:

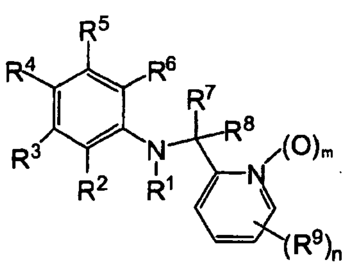

(I)

wherein $R^1$ is alkyl which may be substituted, etc.; each of $R^2$, $R^3$, $R^4$ and $R^6$ which are independent of one another, is hydrogen, halogen, alkyl which may be substituted, etc.; $R^5$ is haloalkyl or halogen; each of $R^7$ and $R^8$ which are independent of each other, is hydrogen, cyano, alkyl, etc.; $R^9$ is alkyl, cycloalkyl, etc.; m is from 0 to 1 and n is from 0 to 4.

EP 2 172 456 A1

## Description

TECHNICAL FIELD

The present invention relates to a novel N-phenyl-methanamine derivative or its salt, and a pesticide containing it as an active ingredient.

BACKGROUND ART

Patent Documents 1 to 4 and Non-Patent Document 1 disclose N-phenyl-methanamine derivatives having certain chemical structures. However, they disclose nothing about application to pesticides. Further, the compounds disclosed in Patent Documents 1 to 3 are different in the chemical structure from the compound of the after-mentioned formula (I), and Patent Document 4 discloses nothing specifically about the compound of the after-mentioned formula (I). On the other hand, Non-Patent Document 1 discloses 2-[bis(2-pyridinylmethyl)amino]-4-chlorophenol in the right column on page 588 and 2-methoxy-5-chlorophenyl-bis(2-pyridylmethyl)amine on the right column on page 594.

Patent Document 1: U.S. Patent Application Publication No. 2004/0224420
Patent Document 2: U.S. Patent Application Publication No. 2003/0008405
Patent Document 3: WO2006/113552
Patent Document 4: WO2003/050174
Non-Patent Document 1: Inorganica Chimica Acta (2000), 300-302 p.587-596

DISCLOSURE OF THE INVENTION

PROBLEMS TO BE SOLVED BY THE INVENTION

For many years, many pesticides have been used, but many of them have various problems such that the effects are inadequate, their use is restricted as pests have acquired resistance, etc. Accordingly, it is desired to develop a novel pesticide substantially free from such problems, for example, a pesticide capable of controlling various pests which create problems in agricultural and horticultural fields or a pesticide capable of controlling pests parasitic on animals.

MEANS TO SOLVE THE PROBLEMS

The present inventors have conducted various studies on N-phenyl-methanamine derivatives in an effort to find a superior pesticide. As a result, they have found that a novel N-phenyl-methanamine derivative has an extremely high pesticidal effect against pests at a low dose and at the same time has safety to crop plants, the natural enemy to pests, or mammals, and have accomplished the present invention.

That is, the present invention relates to an N-phenyl-methanamine derivative represented by the formula (I) or its salt:

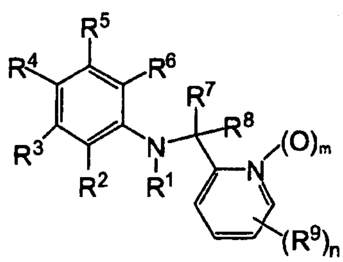

(I)

wherein $R^1$ is alkyl which may be substituted by $R^b$, alkenyl which may be substituted by $R^b$, alkynyl which may be substituted by $R^b$, aryl which may be substituted by $R^d$, or a heterocyclic group which may be substituted by $R^d$; each of $R^2$, $R^3$, $R^4$ and $R^6$ which are independent of one another, is hydrogen, halogen, cyano, isonitrile, nitro, alkyl which may be substituted by $R^b$, alkenyl which may be substituted by $R^b$, alkynyl which may be substituted by $R^b$, cycloalkyl, aryl, a heterocyclic group which may be substituted by alkyl, $NR^aR^c$, $OR^a$, $S(O)_pR^a$, $COR^a$, $COOR^a$ or $CONR^aR^c$; $R^5$ is haloalkyl or halogen; each of $R^7$ and $R^8$ which are independent of each other, is hydrogen, cyano, alkyl, alkenyl, alkynyl, haloalkyl or cycloalkyl, or $R^7$ and $R^8$ may together form $C_{3-6}$ cycloalkyl which may be substituted by halogen; $R^9$ is alkyl, cycloalkyl, alkoxyalkyl, alkoxyalkoxyalkyl, hydroxyalkyl, halogen, haloalkyl, cyano, nitro, aryl which may be substituted

by halogen, a heterocyclic group which may be substituted by halogen, heterocyclic oxy which may be substituted by halogen, $CONR^aR^c$, $COR^a$, $NR^aR^c$, $COOR^a$ or $OR^a$; each of $R^a$ and $R^c$ which are independent of each other, is hydrogen, alkyl, haloalkyl, heterocyclic alkyl, alkoxyalkyl, hydroxyalkyl, cycloalkyl, aryl which may be substituted by $R^d$, or a heterocyclic group which may be substituted by $R^d$; $R^b$ is halogen, aryl which may be substituted by $R^d$, a heterocyclic group which may be substituted by $R^d$, an N-oxide of a nitrogen-containing heterocyclic group which may be substituted by $R^d$, heterocyclic oxy which may be substituted by $R^d$, heterocyclic thio which may be substituted by $R^d$, cyano, $NR^aR^c$, $NHCOOR^a$, $OR^a$, $COR^a$, $COOR^a$ or $S(O)_pR^a$; $R^d$ is alkyl, cycloalkyl, alkoxyalkyl, alkoxyalkoxyalkyl, hydroxyalkyl, halogen, haloalkyl, cyano, nitro, aryl, a heterocyclic group which may be substituted by halogen, heterocyclic oxy which may be substituted by halogen, $COR^a$, amino, monoalkylamino, dialkylamino, $COOR^a$, $OR^a$ or $SR^a$; m is an integer of from 0 to 1; n is an integer of from 0 to 4, p is an integer of from 0 to 2, in the $NR^aR^c$ moiety in each of the above substituents, $R^a$ and $R^c$ may together form a 5- or 6-membered heterocyclic ring together with the nitrogen atom to which they are bonded, provided that 2-[bis(2-pyridinylmethyl)amino]-4-chlorophenol and 2-methoxy-5-chlorophenyl-bis(2-pyridylmethyl)amine are excluded; and a pesticide containing it as an active ingredient.

## EFFECTS OF THE INVENTION

A pesticide containing the N-phenyl-methanamine derivative of the above formula (I) as an active ingredient, has a very high pesticidal effect against pests at a low dose.

## BEST MODE FOR CARRYING OUT THE INVENTION

As the halogen or halogen as the substituent in the formula (I), an atom of fluorine, chlorine, bromine or iodine may be mentioned. The number of halogens as the substituents may be 1 or more, and if more, the respective halogens may be the same or different. Further, the positions for substitution of such halogens may be any positions.

The alkyl or an alkyl moiety in the alkoxy in the formula (I) may be linear or branched. As its specific example, $C_{1-6}$ alkyl such as methyl, ethyl, propyl, isopropyl, butyl, tert-butyl, pentyl or hexyl may be mentioned.

As the cycloalkyl in the formula (I), $C_{3-6}$ cycloalkyl such as cyclopropyl, cyclobutyl, cyclopentyl or cyclohexyl may, for example, be mentioned.

The alkenyl in the formula (I) may be linear or branched. As its specific example, $C_{2-6}$ alkenyl such as vinyl, 1-propenyl, allyl, isopropenyl, 1-butenyl, 1,3-butadienyl or 1-hexenyl may be mentioned.

The alkynyl in the formula (I) may be linear or branched. As its specific example, $C_{2-6}$ alkynyl such as ethynyl, 2-butynyl, 2-pentynyl, 3-methyl-1-butynyl, 2-penten-4-ynyl or 3-hexynyl may be mentioned.

As the aryl in the formula (I), $C_{6-10}$ aryl such as phenyl or naphthyl may, for example, be mentioned.

The heterocyclic group or a heterocyclic moiety in the heterocyclic oxy or the heterocyclic thio in the formula (I) includes a fused heterocyclic group in addition to a monocyclic heterocyclic group. The monocyclic heterocyclic group may, for example, be a 3-membered heterocyclic group such as oxiranyl; a 5-membered heterocyclic group such as furyl, tetrahydrofuryl, thienyl, pyrrolyl, pyrrolinyl, pyrrolidinyl, dioxolanyl, oxazolyl, isoxazolyl, dihydroisoxazolyl, thiazolyl, isothiazolyl, imidazolyl, imidazolinyl, imidazolidinyl, pyrazolyl, pyrazolinyl, pyrazolidinyl, triazolyl, oxadiazolyl, thiadiazolyl, tetrazolyl, 1,3-dioxolanyl, 1,3-oxathiolanyl or 1,3-oxathiolanyl-3-oxide; or a 6-membered heterocyclic group such as pyranyl, pyridyl, piperidinyl, dioxanyl, oxazinyl, morpholinyl, thiazinyl, pyridazinyl, pyrimidinyl, pyrazinyl, piperazinyl, triazinyl, 1,3-dioxanyl, tetrahydropyranyl, 2H-1,4-oxathienyl or 1,3-dithioranyl. Among such monocyclic heterocyclic groups, preferred is a 5- or 6-membered monocyclic heterocyclic group containing from 1 to 4 atoms of at least one type selected from the group consisting of O, S and N. The fused heterocyclic group may, for example, be benzofuranyl, isobenzofuranyl, dihydrobenzofuranyl, dihydroisobenzofuranyl, benzothienyl, isobenzothienyl, dihydrobenzothienyl, dihydroisobenzothienyl, tetrahydrobenzothienyl, indolyl, isoindolyl, benzoxazolyl, benzothiazolyl, indazolyl, benzimidazolyl, benzodioxolanyl, benzodioxanyl, chromenyl, chromanyl, isochromanyl, chromonyl, chromanonyl, quinolyl, isoquinolyl, cinnolinyl, phthalazinyl, quinazolinyl, quinoxalinyl, indolizinyl, quinolizinyl, imidazopyridyl, naphthyridinyl, pteridinyl, dihydrobenzoxazinyl, dihydrobenzoxazolinonyl, dihydrobenzoxazinonyl, benzothioxanyl or imidazopyridinyl. Among such fused heterocyclic groups, preferred is a 8- to 10-membered fused heterocyclic group containing from 1 to 4 atoms of at least one type selected from the group consisting of O, S and N.

In the $NR^aR^c$ moiety in each of the substituents in the formula (I), $R^a$ and $R^c$ may together form a 5- or 6-membered heterocyclic ring together with the nitrogen atom to which they are bonded. Such a 5- or 6-membered heterocyclic ring may further contain, in addition to the nitrogen atom to which $R^a$ and $R^c$ are bonded, at least one hetero atom. Such a heterocyclic ring may, for example, be pyrrolidinyl, pyrazolidinyl, piperazinyl or morpholinyl. Further, the $C_{3-6}$ cycloalkyl which may be substituted by halogen, to be formed by $R^7$ and $R^8$ in the formula (I) may, for example, be cyclopropyl, cyclobutyl, cyclopentyl or cyclohexyl.

The salt of the N-phenyl-methanamine derivative represented by the above formula (I) includes all kinds so long as they are agriculturally acceptable. For example, an alkali metal salt such as a sodium salt or a potassium salt;

an alkaline earth metal salt such as a magnesium salt or a calcium salt; an ammonium salt such as a dimethylammonium salt or a triethylammonium salt; an inorganic acid salt such as a hydrochloride, a perchlorate, a sulfate or a nitrate; or an organic acid salt such as an acetate or a methanesulfonate, may be mentioned.

The N-phenyl-methanamine derivative represented by the above formula (I) may have isomers such as optical isomers or geometrical isomers, and such isomers and mixtures thereof are both included in the present invention. In the present description, isomers are disclosed as mixtures, unless otherwise specified. Further, in the present invention, various isomers other than those mentioned above, may be included within the scope of the common knowledge in this technical field. Further, depending upon the type of such an isomer, the chemical structure may be different from the above-mentioned formula (I), but it is obvious to one skilled in the art that such a structure is in isomeric relation and thus falls within the scope of the present invention.

The N-phenyl-methanamine derivative represented by the above formula (I) or its salt can be produced by the following production processes [1] to [11] and in accordance with a usual method for producing a salt.

PRODUCTION PROCESS [1]

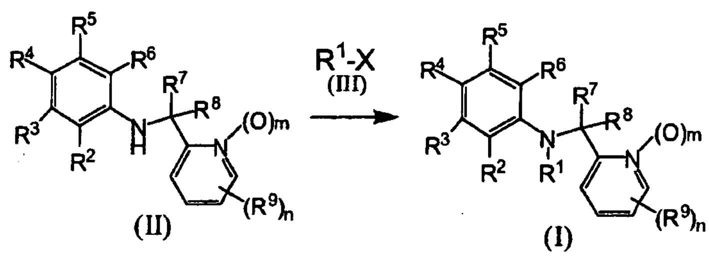


$R^1$, $R^2$, $R^3$, $R^4$, $R^5$, $R^6$, $R^7$, $R^8$, $R^9$, m and n are as defined above; and X is halogen.

The reaction for the production process [1] can be carried out in the presence of a base and a solvent.

The base may, for example, be an alkali metal hydride such as lithium hydride, sodium hydride or potassium hydride; an alkali metal hydroxide such as sodium hydroxide or potassium hydroxide; an alkali metal such as lithium, sodium or potassium; an alkali metal alkoxide such as sodium methoxide, sodium ethoxide or potassium tertiary butoxide; an alkali metal carbonate such as lithium carbonate, sodium carbonate or potassium carbonate; an alkali metal hydrogencarbonate such as lithium hydrogencarbonate, sodium hydrogencarbonate or potassium hydrogencarbonate; or an organic base such as triethylamine or pyridine. The base may be used in an amount of from 1 to 3 equivalents, preferably from 1 to 1.5 equivalents to 1 mol of the compound of the formula (II).

The solvent may be any solvent so long as it is inert to the reaction. For example, it may be an alcohol such as methanol, ethanol, propanol or butanol; an aromatic hydrocarbon such as benzene, toluene or xylene; an aliphatic hydrocarbon such as pentane, hexane, heptane, petroleum ether, ligroin or petroleum benzine; an ether such as diethyl ether, dipropyl ether, dibutyl ether, tetrahydrofuran or dioxane; an ester such as methyl acetate or ethyl acetate; a nitrile such as acetonitrile or propionitrile; an acid amide such as N,N-dimethylformamide or dimethylacetamide; a sulfoxide such as dimethylsulfoxide; a sulfone such as sulfolane; a phosphoric acid amide such as hexamethylphosphoramide; a halogenated hydrocarbon such as chloroform, dichloromethane, carbon tetrachloride or 1,2-dichloroethane; or a mixed solvent thereof.

In the reaction for the production process [1], the compound of the formula (III) can be used in a proportion of from 0.8 to 2 mol to 1 mol of the compound of the formula (II).

The reaction for the production process [1] is carried out usually at a reaction temperature of from 0 to 100˚C, preferably from 0 to 70˚C. The reaction time is usually from 0.1 to 24 hours, preferably from 0.1 to 5 hours.

Various conditions for the reaction in the production process [1] may suitably mutually be combined. Further, among these conditions for the reaction, there are reaction conditions of usual ranges and reaction conditions of preferred ranges, and they may also suitably mutually be selected and combined. PRODUCTION PROCESS [2]

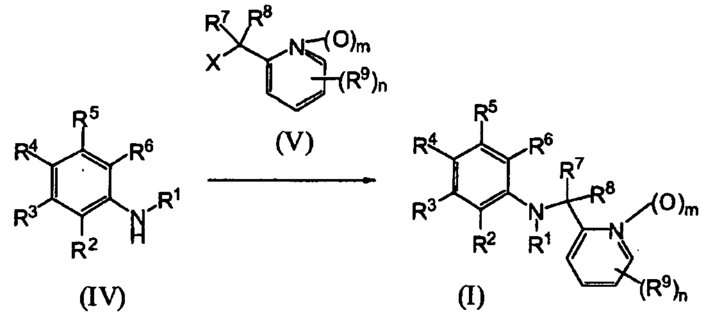

(V)

(IV) (I)

$R^1$, $R^2$, $R^3$, $R^4$, $R^5$, $R^6$, $R^7$, $R^8$, $R^9$, m, n and X are as defined above.

The reaction for the production process [2] can be carried out in the presence of a base and a solvent.

The base may, for example, be the same one as in the above production process [1]. The base may be used in an amount of from 1 to 3 equivalents, preferably from 1 to 1.5 equivalents, to 1 mol of the compound of the formula (IV).

The solvent may be any solvent so long as it is inert to the reaction, and it may, for example, be the same one as in the above production process [1].

In the reaction for the production process [2], the compound of the formula (V) can be used in a proportion of from 0.8 to 2 mol to 1 mol of the compound of the formula (IV).

The reaction for the production process [2] is carried out usually at a reaction temperature of from 0 to 100°C, preferably from 0 to 70°C. The reaction time is usually from 0.1 to 24 hours, preferably from 0.1 to 5 hours.

Various conditions for the reaction in the production process [2] may suitably mutually be combined. Further, among these various conditions for the reaction, there are reaction conditions of usual ranges and reaction conditions of preferred ranges, and they may also suitably mutually be selected and combined.

PRODUCTION PROCESS [3]

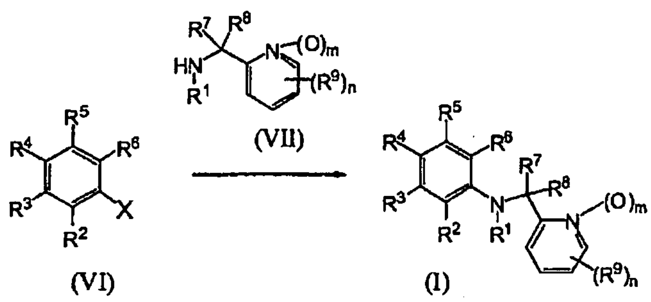

(VII)

(VI) (I)

$R^1$, $R^2$, $R^3$, $R^4$, $R^5$, $R^6$, $R^7$, $R^8$, $R^9$, m, n and X are as defined above.

The reaction for the production process [3] can be carried out in the presence of a solvent.

The solvent may be any solvent so long as it is inert to the reaction, and it may, for example, be the same one as in the above production process [1].

In the reaction for the production process [3], in order to carry out the reaction efficiently, the reaction may be carried out in the presence of a base, as the case requires. Such a base may, for example, be the same one as in the above production process [1].

In the reaction for the production process [3], the compound of the formula (VII) may be used in a proportion of from 0.8 to 5 mol, preferably from 1 to 2.5 mol, to 1 mol of the compound of the formula (VI).

The reaction for the production process [3] is carried out at a reaction temperature of usually from 0 to 150°C, preferably from 0 to 100°C. The reaction time is usually from 0.5 to 100 hours.

Various conditions for the reaction in the production process [3] may suitably mutually be combined. Further, among these conditions for the reaction, there are reaction conditions of usual ranges and reaction conditions of preferred ranges, but they may also suitably mutually be selected and combined.

PRODUCTION PROCESS [4]

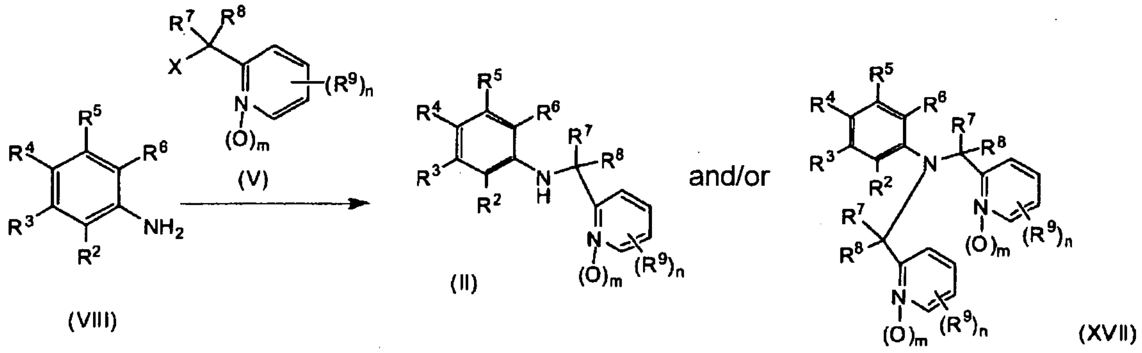

(VIII) (V) (II) and/or (XVII)

$R^2$, $R^3$, $R^4$, $R^5$, $R^6$, $R^7$, $R^8$, $R^9$, m, n and X are as defined above.
The reaction for the production process [4] can be carried out in the presence of a base and a solvent.
The base may, for example, be the same one as in the above production process [1]. The base may be used in an amount of from 1 to 3 equivalents to 1 mol of the compound of the formula (VIII). To obtain the compound of the formula (II), the base is preferably used in an amount of from 1 to 1.5 equivalents, and to obtain the compound of the formula (XVII), the base is preferably used in an amount of from 2 to 2.5 equivalents.
The solvent may be any solvent so long as it is inert to the reaction, and it may, for example, be the same one as in the above production process [1].
In the reaction for the production process [4], the compound of the formula (V) may be used in a proportion of from 0.8 to 2.5 mol to 1 mol of the compound of the above formula (VIII). To obtain the compound of the formula (II), the compound of the formula (V) is preferably used in an amount of from 0.8 to 1.5 mol, and to obtain the compound of the formula (XVII), the compound of the formula (V) is preferably used in an amount of from 2 to 2.5 mol.
The reaction for the production process [4], is carried out at a reaction temperature of usually from 0 to 100˚C, preferably from 0 to 50˚C. The reaction time is usually from 0.5 to 24 hours.
Various conditions for the reaction in the production process [4] may suitably mutually be combined. Further, among these conditions for the reaction, there are reaction conditions of usual ranges and reaction conditions of preferred ranges, but they may also suitably mutually be selected and combined.

PRODUCTION PROCESS [5]

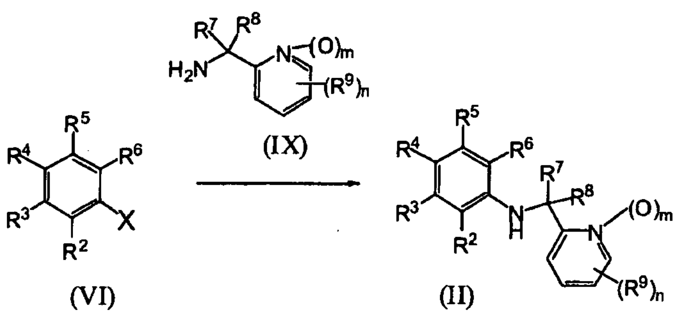

(IX) (VI) (II)

$R^2$, $R^3$, $R^4$, $R^5$, $R^6$, $R^7$, $R^8$, $R^9$, m, n and X are as defined above.
The reaction for the production process [5] can be carried out in the presence of a solvent.
The solvent may be any solvent so long as it is inert to the reaction, and it may, for example, be the same one as in the above production process [1].
In the reaction for the production process [5], in order to carry out the reaction efficiently, the reaction may be carried out in the presence of a base, as the case requires. Such a base may, for example, be the same one as in the above production process [1].

In the reaction for the production process [5], the compound of the formula (IX) may be used in a proportion of from 0.8 to 5 mol, preferably from 1 to 2.5 mol, to 1 mol of the compound of the formula (VI).

The reaction for the production process [5] is carried out at a reaction temperature of usually from 0 to 150˚C, preferably from 0 to 100˚C. The reaction time is usually from 0.5 to 100 hours.

Various conditions for the reaction in the production process [5] may suitably mutually be combined. Further, among these conditions for the reaction, there are reaction conditions of usual ranges and reaction conditions of preferred ranges, but they may also suitably mutually be selected and combined.

PRODUCTION PROCESS [6]

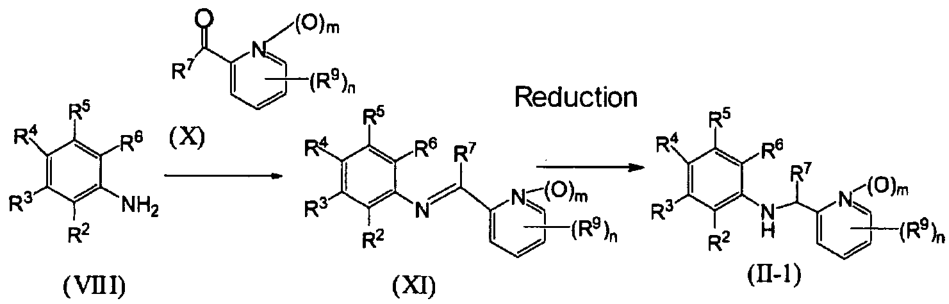


(X)

(VIII) (XI) (II-1)

$R^2$, $R^3$, $R^4$, $R^5$, $R^6$, $R^7$, $R^9$, m and n are as defined above.

The compound of the formula (XI) can be produced by subjecting the compound of the formula (VIII) and the compound of the formula (X) to a condensation reaction in a solvent.

The solvent may be any solvent so long as it is inert to the reaction, and it may, for example, be an alcohol such as methanol, ethanol, propanol or butanol; an aromatic hydrocarbon such as benzene, toluene or xylene; an aliphatic hydrocarbon such as pentane, hexane, heptane, petroleum ether, ligroin or petroleum benzine; an ether such as diethyl ether, dipropyl ether, dibutyl ether, tetrahydrofuran or dioxane; an ester such as methyl acetate or ethyl acetate; an acid amide such as N,N-dimethylformamide or dimethylacetamide; a sulfoxide such as dimethyl sulfoxide; a sulfone such as sulfolane; a phosphoric acid amide such as hexamethylphosphoramide; a halogenated hydrocarbon such as chloroform, dichloromethane, carbon tetrachloride or 1,2-dichloroethane; or a mixed solvent thereof.

In order to carry out the condensation reaction efficiently, an acid catalyst may be used as the case requires. The acid catalyst may, for example, be an inorganic acid such as hydrochloric acid or sulfuric acid; or an organic acid such as acetic acid, camphor sulfonic acid, p-toluene sulfonic acid or pyridinium p-toluene sulfonate.

In the condensation reaction, the compound of the formula (X) may be used in a proportion of from 1 to 2 mol, preferably from 1.2 to 1.5 mol, to 1 mol of the compound of the formula (VIII).

The condensation reaction is carried out at a reaction temperature of usually from 0 to 150˚C, preferably from 50 to 120˚C. The reaction time is usually from 5 to 100 hours.

Various conditions for the condensation reaction may suitably mutually be combined. Further, among such various conditions for the reaction, there are reaction conditions of usual ranges and reaction conditions of preferred ranges, but they may also suitably mutually be selected and combined.

The reduction reaction can be carried out by reacting the compound of the formula (XI) with a reducing agent in a solvent.

The reducing agent may, for example, be a metal hydride such as lithium aluminum hydride, sodium borohydride or sodium cyanoborohydride; or a hydrosilane such as triethylsilane or trichlorosilane. Further, it is also possible to select catalytic reduction, or a method of employing ammonium formate as a reducing agent in Leuckart-Wallach reaction.

The solvent may be any solvent so long as it is inert to the reaction, and it may, for example, be the same one as in the above condensation reaction.

The reduction reaction is carried out at a temperature of usually from 0 to 100˚C, preferably from 0 to 40˚C. The reaction time is usually from about 0.5 to 40 hours.

Various conditions for the reduction reaction may suitably mutually be combined. Further, among such various conditions for the reaction, there are reaction conditions of usual ranges and reaction conditions of preferred ranges, and they may also suitably mutually be selected and combined.

Among compounds of the formula (II) which can be produced by the above production processes [4], [5] or [6], novel compounds are included, and those showing pesticidal activities are included.

PRODUCTION PROCESS [7]

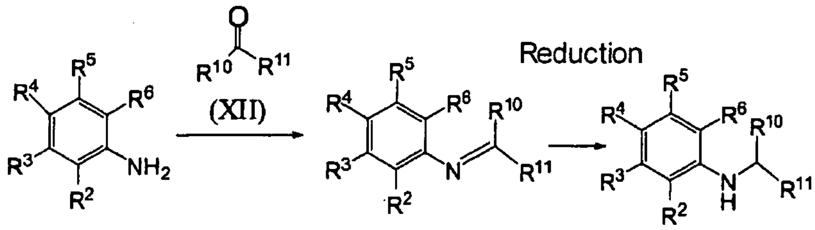


(VIII) (XIII) (XIV)

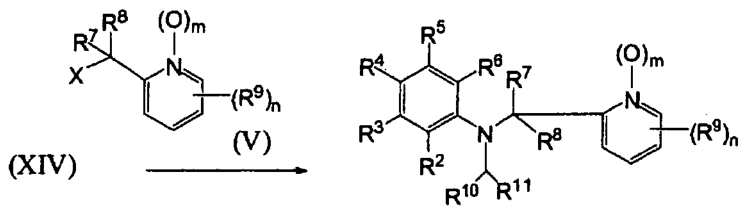

(XIV) (V)

(XV)

Each of $R^{10}$ and $R^{11}$ which are independent of each other, is hydrogen, alkyl, alkenyl, alkynyl, aryl which may be substituted by $R^d$, a heterocyclic group which may be substituted by $R^d$, or an N-oxide of a nitrogen-containing heterocyclic group which may be substituted by $R^d$, and $R^2$, $R^3$, $R^4$, $R^5$, $R^6$, $R^7$, $R^9$, $R^d$, m, n and X are as defined above.
The compound of the formula (XIII) can be produced by subjecting the compound of the formula (VIII) and the compound of the formula (XII) to a condensation reaction in a solvent. The condensation reaction can be carried out in the same manner as the condensation reaction in the above production process [6].
The reduction reaction can be carried out in the same manner as the reduction reaction in the above production process [6].
The compound of the formula (XIV) obtained by the reduction reaction can be reacted with the compound of the formula (V) in the presence of a base and a solvent to obtain a compound of the formula (XV). This reaction can be carried out in the same manner as in the above production process [2].

PRODUCTION PROCESS [8]

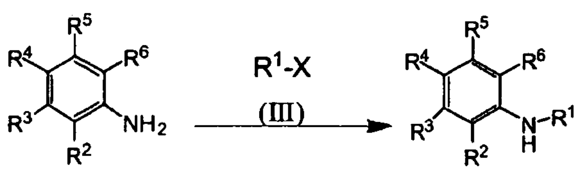


(VIII) (IV)

$R^1$, $R^2$, $R^3$, $R^4$, $R^5$, $R^6$ and X are as defined above.
The reaction of the production process [8] can be carried out in the presence of a solvent.
The solvent may be any solvent so long as it is inert to the reaction, and it may, for example, be the same one as in the above production process [1].
In the reaction for the production process [8], in order to carry out the reaction efficiently, the reaction may be carried out in the presence of a base, as the case requires. Such a base may, for example, be the same one as in the above production process [1].
In the reaction for the production process [8], the compound of the formula (III) may be used in a proportion of from 0.8 to 5 mol, preferably from 1 to 2.5 mol, to 1 mol of the compound of the formula (VIII).
The reaction for the production process [8] is carried out at a reaction temperature of usually from 0 to 150°C,

preferably from 0 to 120°C. The reaction time is usually from 0.5 to 100 hours.

Various conditions for the reaction in the production process [8] may suitably mutually be combined. Further, among these conditions for the reaction, there are reaction conditions of usual ranges and reaction conditions of preferred ranges, but they may also suitably mutually be selected and combined.

PRODUCTION PROCESS [9]

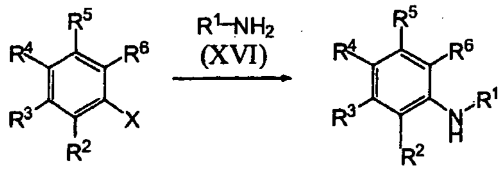


(VI) (IV)

$R^1$, $R^2$, $R^3$, $R^4$, $R^5$, $R^6$ and X are as defined above.

The reaction of the production process [9] can be carried out in the presence of a solvent.

The solvent may be any solvent so long as it is inert to the reaction, and it may, for example, be the same one as in the above production process [1].

In the reaction for the production process [9], in order to carry out the reaction efficiently, the reaction may be carried out in the presence of a base, as the case requires. Such a base may, for example, be the same one as in the above production process [1].

In the reaction for the production process [9], the compound of the formula (XVI) may be used in a proportion of from 0.8 to 5 mol, preferably from 1 to 2.5 mol, to 1 mol of the compound of the formula (VI).

The reaction for the production process [9] is carried out at a reaction temperature of usually from 0 to 150°C, preferably from 0 to 100°C. The reaction time is usually from 0.5 to 100 hours.

Various conditions for the reaction in the production process [9] may suitably mutually be combined. Further, among these conditions for the reaction, there are reaction conditions of usual ranges and reaction conditions of preferred ranges, but they may also suitably mutually be selected and combined.

PRODUCTION PROCESS [10]

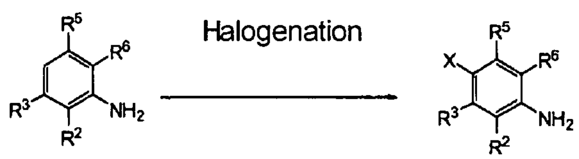


(VIII-1) (VIII-2)

$R^2$, $R^3$, $R^5$, $R^6$ and X are as defined above.

The reaction of the production process [10] can be carried out by using a halogenating agent in the presence of a solvent.

The halogenating agent may, for example, be a chlorinating agent such as chlorine or N-chlorosuccinimide; a brominating agent such as bromine, N-bromosuccinimide or phenyltrimethylammonium tribromide; or an iodizing agent such as iodine or N-iodosuccinimide.

The solvent may be any solvent so long as it is inert to the reaction, and it may, for example, be an aromatic hydrocarbon such as benzene, toluene, xylene or chlorobenzene; an aliphatic hydrocarbon such as carbon tetrachloride, methyl chloride, chloroform, dichloromethane, dichloroethane, trichloroethane, hexane or cyclohexane; an ether such as dioxane, tetrahydrofuran, diethyl ether or dimethoxyethane; an ester such as ethyl acetate; a polar aprotic solvent

such as dimethylsulfoxide, sulfolane, dimethylacetamide, N,N-dimethylformamide, N-methylpyrrolidone or pyridine; an organic acid such as acetic acid or propionic acid; water; or a mixed solvent thereof.

The reaction for the production process [10] is carried out at a reaction temperature of usually from 0 to 150°C, preferably from 20 to 100°C. The reaction time is usually from 0.5 to 24 hours, preferably from 0.5 to 12 hours.

Various conditions for the reaction in the production process [10] may suitably mutually be combined. Further, among these conditions for the reaction, there are reaction conditions of usual ranges and reaction conditions of preferred ranges, but they may also suitably mutually be selected and combined.

PRODUCTION PROCESS [11]

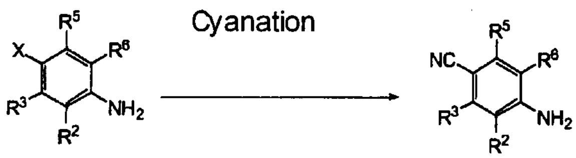


(VIII-2) (VIII-3)

$R^2$, $R^3$, $R^5$, $R^6$ and X are as defined above.

The reaction of the production process [11] can be carried out by using a cyanating agent in the presence of a solvent.

The cyanating agent may, for example, be copper cyanide, zinc cyanide, sodium cyanide, trimethylsilyl cyanide or tributyltin cyanide, and among them, copper cyanide is preferred.

The solvent may be any solvent so long as it is inert to the reaction, and it may, for example, be the same one as in the above production process [1].

In the reaction for the production process [11], the cyanating agent may be used in a proportion of from 0.8 to 5 mol, preferably from 1 to 2.5 mol, to 1 mol of the compound of the formula (VIII-2).

The reaction for the production process [11] is carried out at a reaction temperature of usually from 80 to 200°C, preferably from 100 to 150°C. The reaction time is usually from 1 to 24 hours.

Various conditions for the reaction in the production process [11] may suitably mutually be combined. Further, among these conditions for the reaction, there are reaction conditions of usual ranges and reaction conditions of preferred ranges, but they may also suitably mutually be selected and combined.

Preferred embodiments of pesticides containing the compounds of the present invention will be described below. The pesticides containing the compounds of the present invention are particularly useful, for example, as agents for controlling various pests which become problematic in the agricultural and horticultural fields, i.e. agricultural and horticultural pesticides, or as agents for controlling pests which are parasitic on animals, i.e. pesticides against parasites on animals. Further, the compounds of the formula (II) are also useful as active ingredients for pesticides for controlling various pests which become problematic in the agricultural and horticultural fields, or for controlling pests which are parasitic on animals.

The agricultural and horticultural pesticides are useful as an insecticide, a miticide, a nematicide or a soil pesticide, and they are effective for controlling plant parasitic mites such as two-spotted spider mite (Tetranychus urticae), carmine spider mite (Tetranychus cinnabarinus), kanzawa spider mite (Tetranychus kanzawai), citrus red mite (Panonychus citri), European red mite (Panonychus ulmi), broad mite (Polyphagotarsonemus latus), pink citrus rust mite (Aculops pelekassi) and bulb mite (Rhizoglyphus echinopus); aphids such as green peach aphid (Myzus persicae) and cotton aphid (Aphis gossypii); agricultural insect pests such as diamondback moth (Plutella xylostella), cabbage armyworm (Mamestra brassicae), common cutworm (Spodoptera litura), codling moth (Laspeyresia pomonella), bollworm (Heliothis zea), tobacco budworm (Heliothis virescens), gypsy moth (Lymantria dispar), rice leafroller (Cnaphalocrocis medinalis), Adoxophyes sp., colorado potato beetle (Leptinotarsa decemlineata), cucurbit leaf beetle (Aulacophora femoralis), boll weevil (Anthonomus grandis), planthoppers, leafhoppers, scales, bugs, whiteflies, thrips, grasshoppers, anthomyiid flies, scarabs, black cutworm (Agrotis ipsilon), cutworm (Agrotis segetum) and ants; plant parasitic nematodes such as root-knot nematodes, cyst nematodes, root-lesion nematodes, rice white-tip nematode (Aphelenchoides besseyi), strawberry bud nematode (Nothotylenchus acris), pine wood nematode (Bursaphelenchus lignicolus); gastropods such as slugs and snails; soil pests such as isopods such as pillbugs (Armadilidium vulgare) and pillbugs (Porcellio scaber); hygienic insect pests such as tropical rat mite (Ornithonyssus bacoti), cockroachs, housefly (Musca domestica)

and house mosquito (Culex pipiens); stored grain insect pests such as angoumois grai moth (Sitotroga cerealella), adzuki bean weevil (Callosobruchus chinensis), red flour beetle (Tribolium castaneum) and mealworms; household goods insect pests such as casemaking clothes moth (Tinea pellionella) and black carpet beetle (Anthrenus scrophularidae); house insects pests such as termites; domestic mites such as mold mite (Tyrophagus putrescentiae), Dermatophagoides farinae, Chelacaropsis moorei, and so on. Among them, the agricultural and horticultural pesticides containing the compounds of the present invention are particularly effective for controlling plant parasitic mites, agricultural insect pests, plant parasitic nematodes or the like. Further, they are effective against insect pests having acquired resistance to organophosphorus, carbamate and/or synthetic pyrethroid insecticides. Moreover, the compounds of the present invention have excellent systemic properties, and by the application of the agricultural and horticultural pesticides containing the compounds of the present invention to soil treatment, not only noxious insects, noxious mites, noxious nematodes, noxious gastropods and noxious isopods in soil but also foliage pests can be controlled.

Another preferred embodiments of the pesticides containing compounds of the present invention may be agricultural and horticultural pesticides which collectively control the above-mentioned plant parasitic mites, agricultural insect pests, plant parasitic nematodes, gastropods and soil pests.

The agricultural and horticultural pesticide containing the compound of the present invention, is usually formulated by mixing the compound with various agricultural adjuvants and used in the form of a formulation such as a dust, granules, water-dispersible granules, a wettable powder, a water-based suspension concentrate, an oil-based suspension concentrate, water soluble granules, an emulsifiable concentrate, a soluble concentrate, a paste, an aerosol or an ultra low-volume formulation. However, so long as it is suitable for the purpose of the present invention, it may be formulated into any type of formulation which is commonly used in this field. Such agricultural adjuvants include solid carriers such as diatomaceous earth, slaked lime, calcium carbonate, talc, white carbon, kaoline, bentonite, a mixture of kaolinite and sericite, clay, sodium carbonate, sodium bicarbonate, mirabilite, zeolite and starch; solvents such as water, toluene, xylene, solvent naphtha, dioxane, acetone, isophorone, methyl isobutyl ketone, chlorobenzene, cyclohexane, dimethyl sulfoxide, N,N-dimethylformamide, dimethylacetamide, N-methyl-2-pyrrolidone, and alcohol; anionic surfactants and spreaders such as a salt of fatty acid, a benzoate, an alkylsulfosuccinate, a dialkylsulfosuccinate, a polycarboxylate, a salt of alkylsulfuric acid ester, an alkyl sulfate, an alkylaryl sulfate, an alkyl diglycol ether sulfate, a salt of alcohol sulfuric acid ester, an alkyl sulfonate, an alkylaryl sulfonate, an aryl sulfonate, a lignin sulfonate, an alkyldiphenyl ether disulfonate, a polystyrene sulfonate, a salt of alkylphosphoric acid ester, an alkylaryl phosphate, a styrylaryl phosphate, a salt of polyoxyethylene alkyl ether sulfuric acid ester, a polyoxyethylene alkylaryl ether sulfate, a salt of polyoxyethylene alkylaryl ether sulfuric acid ester, a polyoxyethylene alkyl ether phosphate, a salt of polyoxyethylene alkylaryl phosphoric acid ester, and a salt of a condensate of naphthalene sulfonate with formalin; nonionic surfactants and spreaders such as a sorbitan fatty acid ester, a glycerin fatty acid ester, a fatty acid polyglyceride, a fatty acid alcohol polyglycol ether, acetylene glycol, acetylene alcohol, an oxyalkylene block polymer, a polyoxyethylene alkyl ether, a polyoxyethylene alkylaryl ether, a polyoxyethylene styrylaryl ether, a polyoxyethylene glycol alkyl ether, a polyethylene glycol, a polyoxyethylene fatty acid ester, a polyoxyethylene sorbitan fatty acid ester, a polyoxyethylene glycerin fatty acid ester, a polyoxyethylene hydrogenated castor oil, and a polyoxypropylene fatty acid ester; vegetable and mineral oils such as olive oil, kapok oil, castor oil, palm oil, camellia oil, coconut oil, sesame oil, corn oil, rice bran oil, peanut oil, cottonseed oil, soybean oil, rapeseed oil, linseed oil, tung oil, and liquid paraffins; and so on. Each of the components as such adjuvants may be one or more suitably selected for use, so long as the purpose of the present invention can thereby be accomplished. Further, other than the above-mentioned adjuvants, some among those known in this field may suitably selected for use. The various adjuvants which are commonly used, such as a filler, a thickener, an anti-settling agent, an anti-freezing agent, a dispersion stabilizer, a phytotoxicity reducing agent, an anti-mold agent, and so on, may also be employed.

The weight ratio of the compound of the present invention to the various agricultural adjuvants is from 0.001: 99.999 to 95:5, preferably from 0.005:99.995 to 90:10.

In the actual application of such a formulation, it may be used as it is, or may be diluted to a predetermined concentration with a diluent such as water, and various spreaders e.g. surfactants, vegetable oils or mineral oils may be added thereto, as the case requires.

The application of the agricultural and horticultural pesticide containing the compound of the present invention cannot generally be defined, as it varies depending upon the weather conditions, the type of the formulation, the application season, the application site or the types or degree of outbreak of the pest insects. However, it is usually applied in a concentration of the active ingredient being from 0.05 to 800,000 ppm, preferably from 0.5 to 500,000 ppm, and the dose per unit area is such that the compound of the present invention is from 0.05 to 50,000 g, preferably from 1 to 30,000 g, per hectare. Further, agricultural and horticultural pesticides as another preferred embodiment of pesticides containing the compounds of the present invention may be applied in accordance with the above-described application of pesticides. The present invention includes such a method for controlling pests, particularly for controlling plant parasitic mites, agricultural insect pests or plant parasitic nematodes by such applications.

Various formulations of agricultural and horticultural pesticides containing the compounds of the present invention or their diluted compositions may be applied by conventional methods for application which are commonly employed, such as spraying (e.g. spraying, jetting, misting, atomizing, powder or grain scattering or dispersing in water), soil application (e.g. mixing or drenching), surface application (e.g. coating, powdering or covering) or impregnation to obtain poisonous feed. Further, it is possible to feed domestic animals with a food containing the above active ingredient and to control the outbreak or growth of pests, particularly insect pests, with their excrements. Furthermore, the active ingredient may also be applied by a so-called ultra low-volume application method. In this method, the composition may be composed of 100% of the active ingredient.

Further, the agricultural and horticultural pesticides containing compounds of the present invention may be mixed with or may be used in combination with other agricultural chemicals, fertilizers or phytotoxicity-reducing agents, whereby synergistic effects or activities may sometimes be obtained. Such other agricultural chemicals include, for example, a herbicide, an insecticide, a miticide, a nematicide, a soil pesticide, a fungicide, an antivirus agent, an attractant, an antibiotic, a plant hormone, a plant growth regulating agent, and so on. Especially, with a mixed pesticide having a compound of the present invention mixed with or used in combination with one or more active compounds of other agricultural chemicals, the application range, the application time, the pesticidal activities, etc. may be improved to preferred directions. The compound of the present invention and the active compounds of other agricultural chemicals may separately be formulated so that they may be mixed for use at the time of application, or they may be formulated together. The present invention includes such a mixed pesticidal composition.

The mixing ratio of the compound of the present invention to the active compounds of other agricultural chemicals can not generally be defined, since it varies depending upon the weather conditions, the types of formulations, the application time, the application site, the types or degree of outbreak of insect pests, etc., but it is usually within a range of from 1:300 to 300:1, preferably from 1:100 to 100:1. Further, the dose for the application is such that the total amount of the active compounds is from 0.1 to 50,000 g, preferably from 1 to 30,000 g, per hectare. The present invention includes a method for controlling pests by an application of such a mixed pesticide composition.

The active compounds of insecticides, miticides, nematicides or soil pesticides in the above-mentioned other agricultural chemicals, include, for example, (by common names, some of them are still in an application stage) organic phosphate compounds such as profenofos, dichlorvos, fenamiphos, fenitrothion, EPN, diazinon, chlorpyrifosmethyl, acephate, prothiofos, fosthiazate, phoshocarb, cadusafos, dislufoton, chlorpyrifos, demeton-S-methyl, dimethoate, methamidophos, imicyafos, isoxathion, isofenphos, ethion, etrimfos, quinalphos, dimethylvinphos, sulprofos, thiometon, vamidothion, pyraclofos, pyridaphenthion, pirimiphos-methyl, propaphos, phosalone, formothion, malathion, tetrachlorvinphos, chlorfenvinphos, cyanophos, trichlorfon, methidathion, phenthoate, ESP, azinphos-methyl, fenthion, heptenophos, methoxychlor, parathion, monocrotophos, parathion-methyl, terbufos, phosphamidon, phosmet and phorate; carbamate compounds such as carbaryl, propoxur, aldicarb, carbofuran, thiodicarb, methomyl, oxamyl, ethiofencarb, pirimicarb, fenobucarb, carbosulfan, benfuracarb, bendiocarb, furathiocarb, isoprocarb, metolcarb, xylylcarb, XMC and fenothiocarb; nereistoxin derivatives such as cartap, thiocyclam, bensultap and thiosultap-sodium; organic chlorine compounds such as dicofol, tetradifon, endosulfan, dienochlor and dieldrin; organic metal compounds such as fenbutatin oxide and cyhexatin; pyrethroid compounds such as fenvalerate, permethrin, cypermethrin, deltamethrin, cyhalothrin, tefluthrin, ethofenprox, fenpropathrin, bifenthrin, cyfluthrin, flucythrinate, fluvalinate, cycloprothrin, lambda-cyhalothrin, pyrethrins, esfenvalerate, tetramethrin, resmethrin, protrifenbute, zeta-cypermethrin, acrinathrin, alpha-cypermethrin, allethrin, gamma-cyhalothrin, theta-cypermethrin, tau-fluvalinate, tralomethrin, profluthrin, beta-cypermethrin, beta-cyfluthrin, metofluthrin, flumethrin and phenothrin; benzoylurea compounds such as diflubenzuron, chlorfluazuron, teflubenzuron, flufenoxuron, lufenuron, novaluron, triflumuron, hexaflumuron, noviflumuron, bistrifluron and fluazuron; juvenile hormone-like compounds such as methoprene, pyriproxyfen, fenoxycarb and diofenolan; pyridazinone compounds such as pyridaben; pyrazole compounds such as fenpyroximate, fipronil, tebufenpyrad, ethiprole, tolfenpyrad, acetoprole, pyrafluprole and pyriprole; neonicotinoids such as imidacloprid, nitenpyram, acetamiprid, thiacloprid, thiamethoxam, clothianidin, dinotefuran and nithiazine; hydrazine compounds such as tebufenozide, methoxyfenozide, chromafenozide and halofenozide; other compounds such as flonicamid, buprofezin, hexythiazox, amitraz, chlordimeform, silafluofen, triazamate, pymetrozine, pyrimidifen, chlorfenapyr, indoxacarb, acequinocyl, etoxazole, cyromazine, 1,3-dichloropropene, diafenthiuron, benclothiaz, flufenrim, pyridalyl, spirodiclofen, bifenazate, spiromesifen, spirotetramat, propargite, clofentezine, fluacrypyrim, metaflumizone, flubendiamide, chlorantraniliprole, cyflumetofen, cyenopyrafen, pyrifluquinazon, fenazaquin, pyridaben, amidoflumet, chlorobenzoate, sulfluramid, hydramethylnon, metaldehyde and ryanodine. Further, microbial agricultural chemicals such as insecticidal crystal protein produced by Bacillus thuringienses aizawai, Bacillus thuringienses kurstaki, Bacillus thuringienses israelensis, Bacillus thuringienses japonensis, Bacillus thuringienses tenebrionis or Bacillus thuringienses, insect viruses, etomopathogenic fungi, and nematophagous fungi; antibiotics or semisynthetic antibiotics such as avermectin, emamectin-benzoate, milbemectin, spinosad, ivermectin, lepimectin, spinetoram, abamectin and emamectin; natural products such as azadirachtin and rotenone; and repellents such as deet may, for example, be mentioned.

The fungicidal active compounds in the above-mentioned other agricultural chemicals include, for example, (by common names, some of them are still in an application stage, or test codes of Japan Plant Protection Association)

anilinopyrimidine compounds such as mepanipyrim, pyrimethanil and cyprodinil; pyridinamine compounds such as fluazinam; azole compounds such as triadimefon, bitertanol, triflumizole, etaconazole, propiconazole, penconazole, flusilazole, myclobutanil, cyproconazole, tebuconazole, hexaconazole, furconazole-cis, prochloraz, metconazole, epoxiconazole, tetraconazole, oxpoconazole fumarate, sipconazole, prothioconazole, triadimenol, flutriafol, difenoconazole, fluquinconazole, fenbuconazole, bromuconazole, diniconazole, tricyclazole, probenazole, simeconazole, pefurazoate, ipconazole and imibenconazole; quinoxaline compounds such as quinomethionate; dithiocarbamate compounds such as maneb, zineb, mancozeb, polycarbamate, metiram, propineb and thiram; organic chlorine compounds such as fthalide, chlorothalonil and quintozene; imidazole compounds such as benomyl, thiophanatemethyl, carbendazim, thiabendazole, fuberiazole and cyazofamid; cyanoacetamide compounds such as cymoxanil; phenylamide compounds such as metalaxyl, metalaxyl-M, mefenoxam, oxadixyl, ofurace, benalaxyl, benalaxyl-M (another name: kiralaxyl, chiralaxyl), furalaxyl and cyprofuram; sulfenic acid compounds such as dichlofluanid; copper compounds such as cupric hydroxide and oxine copper; isoxazole compounds such as hymexazol; organophosphorus compounds such as fosetyl-Al, tolcofos-methyl, S-benzyl O,O-diisopropylphosphorothioate, O-ethyl S,S-diphenylphosphorodithioate, aluminum ethylhydrogen phosphonate, edifenphos and iprobenfos; N-halogenothioalkyl compounds such as captan, captafol and folpet; dicarboximide compounds such as procymidone, iprodione and vinclozolin; benzanilide compounds such as flutolanil, mepronil, zoxamid and tiadinil; anilide compounds such as carboxin, oxycarboxin, thifluzamide, penthiopyrad, boscalid, isothianil and bixafen; piperazine compounds such as triforine; pyridine compounds such as pyrifenox; carbinol compounds such as fenarimol and flutriafol; piperidine compounds such as fenpropidine; morpholine compounds such as fenpropimorph and tridemorph; organotin compounds such as fentin hydroxide and fentin acetate; urea compounds such as pencycuron; cinnamic acid compounds such as dimethomorph and flumorph; phenylcarbamate compounds such as diethofencarb; cyanopyrrole compounds such as fludioxonil and fenpiclonil; strobilurin compounds such as azoxystrobin, kresoximmethyl, metominofen, trifloxystrobin, picoxystrobin, oryzastrobin, dimoxystrobin, pyraclostrobin, fluoxastrobin and fluacrypyrin; oxazolidinone compounds such as famoxadone; thiazolecarboxamide compounds such as ethaboxam; silylamide compounds such as silthiopham; aminoacid amidecarbamate compounds such as iprovalicarb and benthiavalicarb-isopropyl; imidazolidine compounds such as fenamidone; hydroxanilide compounds such as fenhexamid; benzenesulfonamide compounds such as flusulfamide; oxime ether compounds such as cyflufenamid; phenoxyamide compounds such as fenoxanil; antibiotics such as validamycin, kasugamycin and polyoxins; guanidine compounds such as iminoctadine; and other compounds such as isoprothiolane, pyroquilon, diclomezine, quinoxyfen, propamocarb hydrochloride, spiroxamine, chloropicrin, dazomet, metam-sodium, nicobifen, metrafenone, UBF-307, diclocymet, proquinazid, amisulbrom, pyribencarb mandipropamid, fluopicolide, carpropamid, meptyldinocap, fluopyram, BCF051, BCM061 and BCM062.

Further, agricultural chemicals which may be used in admixture with or in combination with the compounds of the present invention, may, for example, be the active ingredient compounds in the herbicides as disclosed in Farm Chemicals Handbook (2002 edition), particularly those of soil treatment type.

The pesticides against parasites on animals are effective for controlling e.g. external parasites which are parasitic on the body surface of host animals (such as the back, the axilla, the lower abdomen or inside of the thigh) or internal parasites which are parasitic in the body of host animals (such as the stomach, the intestinal tract, the lung, the heart, the liver, the blood vessels, the subcutis or lymphatic tissues), but they are particularly effective for controlling the external parasites.

The external parasites may, for example, be animal parasitic acarus or fleas. Their species are so many that it is difficult to list all of them, and therefore, their typical examples will be given.

The animal parasitic acarus may, for example, be ticks such as Boophilus microplus, Rhipicephalus sanguineus, Haemaphysalis longicornis, Haemaphysalis flava, Haemaphysalis campanulata, Haemaphysalis concinna, Haemaphysalis japonica, Haemaphysalis kitaokai, Haemaphysalis ias, Ixodes ovatus, Ixodes nipponensis, Ixodes persulcatus, Amblyomma testudinarium, Haemaphysalis megaspinosa, Dermacentor reticulates, and Dermacentor taiwanesis; common red mite (Dermanyssus qallinae); northern fowl mites such as Ornithonyssus sylviarum, and Ornithonyssus bursa; trombidioids such as Eutrombicula wichmanni, Leptotrombidium akamushi, Leptotrombidium pallidum, Leptotrombidium fuji, Leptotrombidium tosa, Neotrombicula autumnalis, Eutrombicula alfreddugesi, and Helenicula miyagawai; cheyletidae such as Cheyletiella yasguri, Cheyletiella parasitivorax, and Cheyletiella blakei; sarcoptic mange mites such as Psoroptes cuniculi, Chorioptes bovis, Otodectes cynotis, Sarcoptes scabiei, and Notoedres cati; and Demodicidae such as Demodex canis. The pesticides against parasites on animals, containing the compounds of the present invention, are particularly effective for the control of ticks among them.

The fleas may, for example, be externally parasitic wingless insects belonging to Siphonaptera, more specifically, fleas belonging to Pulicidae, Ceratephyllus, etc. Fleas belonging to Pulicidae may for example, be Ctenocephalides canis, Ctenocephalides felis, Pulex irritans, Echidnophaga gallinacea, Xenopsylla cheopis, Leptopsylla segnis, Nosopsyllus fasciatus, and Monopsyllus anisus. The pesticides against parasites on animals, containing the compounds of the present invention, are particularly effective for the control of fleas belonging to Pulicidae, particularly Ctenocephalides canis and Ctenocephalides felis, among them.

Other external parasites may, for example, be sucking lice (Anoplura) such as shortnosed cattle louse (Haematopinus eurysternus), horse sucking louse (Haematopinus asini), sheep louse, longnosed cattle louse (Linognathus vituli), and head louse Pediculus capitis); biting lice such as dog biting louse (Trichodectes canis); and blood-sucking dipterous insects such as horsefly (Tabanus trigonus), biting midges (Culicoides schultzei), and blackfly (Simulium ornatum). Further, the internal parasites may, for example, be nematodes such as lung worms, whipworms Trichuris), tuberous worms, gastric parasites, ascaris, and filarioidea; cestoda such as Spirometra erinacei, Diphyllobothrium latum, Dipylidium caninum, Taenia multiceps, Echinococcus granulosus, Echinococcus multilocularis; trematoda such as Schistosoma japonicum, Fasciola hepatica; and protozoa such as coccidia, malaria parasites (Plasmodium malariae), intestinal sarcocyst, toxoplasma, and cryptosporidium.

The host animals may, for example, be pet animals, domestic animals, and poultry, such as dogs, cats, mice, rats, hamsters, guinea pigs, squirrels, rabbits, ferrets, birds (such as pigeons, parrots, hill mynas, Java sparrows, honey parrots, lovebirds and canaries), cows, horses, pigs, sheep, ducks and chickens. The pesticides against parasites on animals, containing the compounds of the present invention, are particularly effective for the control of pests parasitic on pet animals or domestic animals, especially for the control of external parasites, among them. Among pet animals or domestic animals, they are effective particularly for dogs and cats, cows and horses.

When the compound of the present invention is used as a pesticide against parasites on animals, it may be used as it is or may be used together with suitable adjuvants, as formulated into various formulations such as a dust, granules, tablets, a powder, capsules, a soluble concentrate, an emulsifiable concentrate, a water-based suspension concentrate and an oil-based suspension concentrate. In addition to such formulations, it may be formulated into any type of formulation which is commonly used in this field, so long as it is suitable for the purpose of the present invention. The adjuvants to be used for formulations may, for example, be anionic surfactants or nonionic surfactants exemplified above as adjuvants for formulation of agricultural and horticultural pesticides; a cationic surfactant such as cetyl trimethylammonium bromide; a solvent such as water, acetone, acetonitrile, monomethylacetamide, dimethylacetamide, N,N-dimethylformamide, 2-pyrrolidone, N-methyl-2-pyrrolidone, kerosene, triacetin, methanol, ethanol, isopropanol, benzyl alcohol, ethylene glycol, propylene glycol, polyethylene glycol, liquid polyoxyethylene glycol, butyl diglycol, ethylene glycol monomethyl ether, ethylene glycol monoethyl ether, diethylene glycol monoethyl ether, diethylene glycol n-butyl ether, dipropylene glycol monomethyl ether, or dipropylene glycol n-butyl ether; an antioxidant such as butylhydroxyanisole, butylhydroxytoluene, ascorbic acid, sodium hydrogenmetasulfite, propyl gallate or sodium thiosulfate; a coating film-forming agent such as polyvinylpyrrolidone, polyvinyl alcohol, or a copolymer of vinyl acetate and vinyl pyrrolidone; the vegetable oils and mineral oils as exemplified above as adjuvants for formulation of agricultural and horticultural pesticides; a carrier such as lactose, sucrose, glucose, starch, wheat flour, corn powder, soybean cake and meal, defatted rice bran, calcium carbonate or other commercially available feed materials; and so on. One or more of the respective components of these adjuvants may be suitably selected for use, so long as such will not depart from the purpose of the present invention. Further, other than the above-mentioned adjuvants, some among those known in this field may suitably be selected for use, and still further, some among the above-mentioned various adjuvants to be used in the agricultural and horticultural field may suitably be selected for use.

The blend ratio of the compound of the present invention to various adjuvants is usually from 0.1:99.9 to 90:10. In the actual use of such a formulation, it may be used as it is, or may be diluted to a predetermined concentration with a diluent such as water, and various spreaders (e.g. surfactants, vegetable oils or mineral oils) may be added thereto, as the case requires.

Administration of the compound of the present invention to a host animal is carried out orally or parenterally. As an oral administration method, a method of administering a tablet, a liquid agent, a capsule, a wafer, a biscuit, a minced meat or other feed, containing the compound of the present invention, may be mentioned. As a parenteral administration method, there may, for example, be mentioned a method wherein the compound of the present invention is formulated into a suitable formulation and then taken into the body by e.g. intravenous administration, intramuscular administration, intradermal administration, hypodermic administration, etc.; a method wherein it is administered on the body surface by spot-on treatment, pour-on treatment or spray treatment; or a method of embedding a resin fragment or the like containing the compound of the present invention under the skin of the host animal.

The dose of the compound of the present invention to a host animal varies depending upon the administration method, the purpose of administration, the deceased symptom, etc., but it is usually administered in a proportion of from 0.01 mg to 100 g, preferably from 0.1 mg to 10 g, per 1 kg of the body weight of the host animal.

The present invention also includes a method for controlling a pest by the above-mentioned administration method or by the above-mentioned dose, particularly a method for controlling external parasites or internal parasites.

Further, in the present invention, by controlling pests parasitic on animals as described above, it is possible to prevent or cure various diseases of the host animal thereby caused in some cases. Thus, the present invention also includes a preventive or therapeutic agent for an animal disease caused by parasites, containing the compound of the present invention as an active ingredient, and a method for preventing or curing an animal disease caused by parasites.

When the compound of the present invention is used as a pesticide against parasites on animals, various

vitamins, minerals, amino acids, nutrients, enzymes, antipyretics, sedatives, antiphlogistics, fungicides, colorants, aromatic substances, preservatives, etc., may be used in admixture with or in combination with the adjuvants. Further, as the case requires, other animal drugs or agricultural chemicals, such as vermicides, anti-coccidium agents, insecticides, miticides, pulicides, nematocides, bactericides or antibacterial agents, may be mixed or combined for use, whereby improved effects may sometimes be obtained. The present invention includes such a mixed pesticidal composition having the above-mentioned various components mixed or combined for use, and further a method for controlling a pest by using it, particularly a method for controlling external parasites or internal parasites.

Preferred embodiments of the compound useful as the active ingredient of the pesticide of the present invention will be shown below. However, it should be understood that the present invention is by no means thereby restricted.

(1) The N-phenyl-methanamine derivative of the above formula (I) or its salt, wherein $R^1$ is alkyl which may be substituted by $R^b$; each of $R^2$, $R^3$, $R^4$ and $R^6$ which are independent of one another, is hydrogen, halogen, cyano, nitro, alkyl which may be substituted by $R^b$, $OR^a$ or $S(O)_pR^a$; each of $R^7$ and $R^8$ which are independent of each other, is hydrogen or alkyl; m is 0, n is 0, and p is 0.

(2) The N-phenyl-methanamine derivative of the above formula (I) or its salt, wherein at least one of $R^2$, $R^3$, $R^4$ and $R^6$ is halogen or cyano.

(3) The N-phenyl-methanamine derivative or its salt according to the above (1), wherein at least one of $R^2$, $R^3$, $R^4$ and $R^6$ is halogen, or at least one of $R^3$ and $R^4$ is cyano.

(4) The N-phenyl-methanamine derivative or its salt according to the above (1), wherein each of $R^2$ and $R^6$ which are independent of each other, is hydrogen, halogen or $OR^a$.

(5) The N-phenyl-methanamine derivative or its salt according to the above (1), wherein each of $R^2$ and $R^6$ is hydrogen.

(6) The N-phenyl-methanamine derivative of the above formula (I) or its salt, wherein $R^5$ is haloalkyl.

(7) The N-phenyl-methanamine derivative or its salt according to the above (6), wherein $R^5$ is trifluoromethyl, trichloromethyl, chlorodifluoromethyl, difluoromethyl, perfluoroethyl, perfluoroisopropyl or perfluoro-tert-butyl.

(8) An N-phenyl-methanamine derivative represented by the formula (I) or its salt:

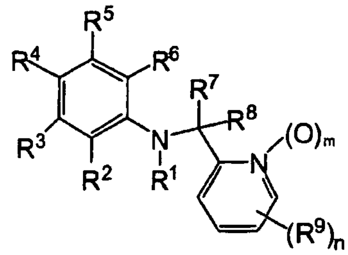

(I)

wherein $R^1$ is alkyl which may be substituted by $R^b$, alkenyl which may be substituted by $R^b$, alkynyl which may be substituted by $R^b$, aryl which may be substituted by $R^d$, or a heterocyclic group which may be substituted by $R^d$; each of $R^2$ and $R^6$ which are independent of each other, is hydrogen, halogen, cyano, nitro, alkyl which may be substituted by $R^b$, alkenyl which may be substituted by $R^b$, alkynyl which may be substituted by $R^b$, $OR^a$, $SR^a$, $NR^aR^c$, $COOR^a$ or $COR^a$; each of $R^3$ and $R^4$ which are independent of each other, is hydrogen, halogen, cyano, isonitrile, nitro, alkyl which may be substituted by $R^b$, cycloalkyl, alkenyl, alkynyl, aryl, a heterocyclic group which may be substituted by alkyl, $NR^aR^c$, $OR^a$, $S(O)_pR^a$, $COR^a$, $COOR^a$ or $CONR^aR^c$; $R^5$ is haloalkyl or halogen; each of $R^7$ and $R^8$ which are independent of each other, is hydrogen, cyano, alkyl, alkenyl, alkynyl, haloalkyl or cycloalkyl, or $R^7$ and $R^8$ may together form $C_{3-6}$ cycloalkyl which may be substituted by halogen; $R^9$ is alkyl, cycloalkyl, alkoxyalkyl, alkoxyalkoxyalkyl, hydroxyalkyl, halogen, haloalkyl, cyano, nitro, aryl which may be substituted by halogen, a heterocyclic group which may be substituted by halogen, heterocyclic oxy which may be substituted by halogen, $CONR^aR^c$, $COR^c$, $NR^aR^c$, $COOR^c$ or $OR^a$; $R^a$ is hydrogen, alkyl, alkoxyalkyl, cycloalkyl, haloalkyl or heterocyclic alkyl; $R^b$ is halogen, aryl which may be substituted by $R^d$, a heterocyclic group which may be substituted by $R^d$, an N-oxide of a nitrogen-containing heterocyclic group which may be substituted by $R^d$, heterocyclic oxy which may be substituted by $R^d$, heterocyclic thio which may be substituted by $R^d$, cyano, $NR^aR^c$, $NHCOOR^a$, $OR^a$, $COR^c$, $COOR^c$ or $S(O)_pR^a$; $R^c$ is hydrogen, alkyl, cycloalkyl, alkoxyalkyl, hydroxyalkyl, aryl may be substituted by $R^d$, or a heterocyclic group which may be substituted by $R^d$; $R^d$ is alkyl, cycloalkyl, alkoxyalkyl, alkoxyalkoxyalkyl, hydroxyalkyl, halogen, haloalkyl, cyano, nitro, aryl, a heterocyclic group which may be substituted by halogen, heterocyclic oxy which may be substituted by halogen, $COR^a$, amino, monoalkylamino, dialkylamino, $COOR^a$, $OR^a$ or $SR^a$; m is an integer of from 0 to 1; n is an integer of from 0 to 4, p is an integer of from 0 to 2, in the $NR^aR^c$ moiety in each of the above substituents, $R^a$ and $R^c$ may together form a 5- or 6-membered heterocyclic ring together with the

nitrogen atom to which they are bonded, provided that 2-[bis(2-pyridinylmethyl)amino]-4-chlorophenol and 2-methoxy-5-chlorophenyl-bis(2-pyridylmethyl)amine are excluded.

(9) The N-phenyl-methanamine derivative or its salt according to the above (8), wherein $R^1$ is an alkyl which may be substituted by $R^b$; each of $R^2$ and $R^6$ which are independent of each other, is hydrogen, halogen, alkyl or $OR^a$; each of $R^3$ and $R^4$ which are independent of each other, is hydrogen, halogen, cyano, nitro, alkyl which may be substituted by $R^b$, $OR^a$ or $S(O)_pR^a$; each of $R^7$ and $R^8$ which are independent of each other, is hydrogen or alkyl, m is 0, n is 0, and p is 0.

(10) An N-phenyl-methanamine derivative represented by the formula (II) or its salt:

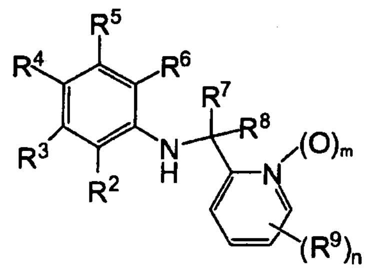

(II)

wherein each of $R^2$, $R^3$, $R^4$ and $R^6$ which are independent of one another, is hydrogen atom, halogen, cyano, isonitrile, nitro, alkyl which may be substituted by $R^b$, alkenyl which may be substituted by $R^b$, alkynyl which may be substituted by $R^b$, cycloalkyl, aryl, a heterocyclic group which may be substituted by alkyl, $NR^aR^c$, $OR^a$, $S(O)_pR^a$, $COR^a$, $COOR^a$ or $CONR^aR^c$; $R^5$ is haloalkyl or halogen; each of $R^7$ and $R^3$ which are independent of each other, is hydrogen atom, cyano, alkyl, alkenyl, alkynyl, haloalkyl or cycloalkyl, or $R^7$ and $R^8$ may together form $C_{3-6}$ cycloalkyl which may be substituted by halogen; $R^9$ is alkyl, cycloalkyl, alkoxyalkyl, alkoxyalkoxyalkyl, hydroxyalkyl, halogen, haloalkyl, cyano, nitro, aryl which may be substituted by halogen, a heterocyclic group which may be substituted by halogen, heterocyclic oxy which may be substituted by halogen, $CONR^aR^c$, $COR^a$, $NR^aR^c$, $COOR^a$ or $OR^a$; each of $R^a$ and $R^c$ which are independent of each other, is hydrogen, alkyl, haloalkyl, heterocyclic alkyl, alkoxyalkyl, hydroxyalkyl, cycloalkyl, aryl which may be substituted by $R^d$, or a heterocyclic group which may be substituted by $R^d$; $R^b$ is halogen, aryl which may be substituted by $R^d$, a heterocyclic group which may be substituted by $R^d$, an N-oxide of a nitrogen-containing heterocyclic group which may be substituted by $R^d$, heterocyclic oxy which may be substituted by $R^d$, heterocyclic thio which may be substituted by $R^d$, cyano, $NR^aR^c$, $NHCOOR^a$, $OR^a$, $COR^a$, $COOR^a$ or $S(O)_pR^a$; $R^d$ is alkyl, cycloalkyl, alkoxyalkyl, alkoxyalkoxyalkyl, hydroxyalkyl, halogen, haloalkyl, cyano, nitro, aryl, a heterocyclic group which may be substituted by halogen, heterocyclic oxy which may be substituted by halogen, $COR^a$, amino, monoalkylamino, dialkylamino, $COOR^a$, $OR^a$ or $SR^a$; m is an integer of from 0 to 1; n is an integer of from 0 to 4, and p is an integer of from 0 to 2, and in the $NR^aR^c$ moiety in each of the above substituents, $R^a$ and $R^c$ may together form a 5- or 6-membered heterocyclic ring together with the nitrogen atom to which they are bonded.

(11) The N-phenyl-methanamine derivative or its salt according to the above (10), wherein each of $R^2$ and $R^6$ which are independent of each other, is hydrogen, halogen, alkyl or $OR^a$; each of $R^3$ and $R^4$ which are independent of each other, is hydrogen, halogen, cyano, nitro, alkyl which may be substituted by $R^b$, $OR^a$ or $S(O)_pR^a$; each of $R^7$ and $R^3$ which are independent of each other, is hydrogen or alkyl, m is 0, n is 0, and p is 0.

(12) The N-phenyl-methanamine derivative or its salt according to the above (11), wherein each of $R^3$ and $R^4$ which are independent of each other, is hydrogen, halogen, nitro, alkyl which may be substituted by $R^b$, $OR^a$ or $S(O)_pR^a$.

(13) An N-phenyl-methanamine derivative represented by the formula (II) or its salt:

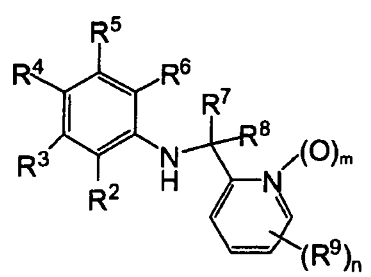

(II)

wherein each of $R^2$ and $R^6$ which are independent of each other, is hydrogen atom, halogen, cyano, nitro, alkyl which

may be substituted by $R^b$, alkenyl which may be substituted by $R^b$, alkynyl which may be substituted by $R^b$, $OR^a$, $SR^a$, $NR^aR^c$, $COOR^a$ or $COR^a$; each of $R^3$ and $R^4$ which are independent of each other, is hydrogen, halogen, cyano, isonitrile, nitro, alkyl which may be substituted by $R^b$, cycloalkyl, alkenyl, alkynyl, aryl, a heterocyclic group which may be substituted by alkyl, $NR^aR^c$, $OR^a$, $S(O)_pR^a$, $COR^a$, $COOR^a$ or $CONR^aR^c$; $R^5$ is haloalkyl or halogen; each of $R^7$ and $R^8$ which are independent of each other, is hydrogen, cyano, alkyl, alkenyl, alkynyl, haloalkyl or cycloalkyl, or $R^7$ and $R^8$ may together form $C_{3-6}$ cycloalkyl which may be substituted by halogen; $R^9$ is alkyl, cycloalkyl, alkoxyalkyl, alkoxyalkoxyalkyl, hydroxyalkyl, halogen, haloalkyl, cyano, nitro, aryl which may be substituted by halogen, a heterocyclic group which may be substituted by halogen, heterocyclic oxy which may be substituted by halogen, $CONR^aR^c$, $COR^c$, $NR^aR^c$, $COOR^c$ or $OR^a$; $R^a$ is hydrogen, alkyl, alkoxyalkyl, cycloalkyl, haloalkyl or heterocyclic alkyl; $R^b$ is halogen, aryl which may be substituted by $R^d$, a heterocyclic group which may be substituted by $R^d$, an N-oxide of a nitrogen-containing heterocyclic group which may be substituted by $R^d$, heterocyclic oxy which may be substituted by $R^d$, heterocyclic thio which may be substituted by $R^d$, cyano, $NR^aR^c$, $NHCOOR^a$, $OR^a$, $COR^c$, $COOR^c$ or $S(O)_pR^a$; $R^c$ is hydrogen, alkyl, cycloalkyl, alkoxyalkyl, hydroxyalkyl, aryl which may be substituted by $R^d$, or a heterocyclic group which may be substituted by $R^d$; $R^d$ is alkyl, cycloalkyl, alkoxyalkyl, alkoxyalkoxyalkyl, hydroxyalkyl, halogen, haloalkyl, cyano, nitro, aryl, a heterocyclic group which may be substituted by halogen, heterocyclic oxy which may be substituted by halogen, $COR^a$, amino, monoalkylamino, dialkylamino, $COOR^a$, $OR^a$ or $SR^a$; m is an integer of from 0 to 1; n is an integer of from 0 to 4, p is an integer of from 0 to 2, and in the $NR^aR^c$ moiety in each of the above substituents, $R^a$ and $R^c$ may together form a 5- or 6-membered heterocyclic ring together with the nitrogen atom to which they are bonded.

Now, with respect to the method for controlling a pest, which comprises applying an effective amount of the N-phenyl-methanamine derivative of the above formula (I) or its salt, preferred embodiments will be shown below. However, it should be understood that the present invention is by no means thereby restricted.

(1) A method for controlling an animal parasite, which comprises applying an effective amount of the N-phenyl-methanamine derivative of the above formula (I) or its salt to the animal parasite.
(2) The method as defined in the above (1), wherein the animal parasite is an external parasite parasitic on a host animal.
(3) The method according to the above (2), wherein the external parasite on a host animal is an animal parasitic acarus or flea.
(4) The method according to the above (3), wherein the animal parasitic acarus is ticks, northern fowl mites, trombidioids, cheyletidae, sarcoptic mange mites or Demodicidae.
(5) The method according to the above (3), wherein the animal parasitic acarus is ticks.
(6) The method according to the above (3), wherein the flea is a flea belonging to Pulicidae or Ceratephyllus.
(7) The method according to the above (6), wherein the flea belonging to Pulicidae is Ctenocephalides canis, Ctenocephalides felis, Pulex irritans, Echidnophaga gallinacea, Xenopsylla cheopis, Leptopsylla segnis, Nosopsyllus fasciatus, or Monopsyllus anisus.
(8) The method according to the above (6), wherein the flea belonging to Pulicidae is Ctenocephalides canis or Ctenocephalides felis.
(9) The method according to the above (2), wherein the external parasite on a host animal is sucking lice, biting lice or blood-sucking dipterous insect.
(10) The method according to the above (2), wherein the host animal is a pet animal, a domestic animal or a poultry.

EXAMPLES

Now, the present invention will be described in further detail with reference to Examples, but it should be understood that the present invention is by no means thereby restricted. Firstly, Preparation Examples of the compound of the present invention will be described.

PREPARATION EXAMPLE 1

Preparation of N-4-chloro-3,5-bis(trifluoromethyl)phenyl)-N-(2-pyridinylmethyl)-2-pyridine methanamine (compound No. 27)

(1) 1.0 g of 3,5-bis(trifluoromethyl)aniline was dissolved in 3 mL of N,N-dimethylformamide, and 640 mg of N-chlorosuccinimide was added and reacted at about 50˚C for about one hour, whereupon the reaction solution was left to cool. To the reaction solution, water was added and stirred, and it was extracted with ethyl acetate. The crude product thereby obtained was purified by silica gel column chromatography (eluent: n-hexane/ethyl acetate=4/1) to

obtain 120 mg of 4-chloro-3,5-bis(trifluoromethyl)aniline.

(2) 120 mg of 4-chloro-3,5-bis(trifluoromethyl)aniline was dissolved in 2 mL of N,N-dimethylformamide, and 240 mg of 2-bromomethylpyridine hydrogenbromide and then 80 mg of sodium hydride were added and reacted at room temperature for about one hour. To the reaction solution, water was added and stirred, and then, it was extracted with ethyl acetate. Then, the crude product thereby obtained was purified by silica gel column chromatography (eluent: ethyl acetate) to obtain 120 mg of the desired product (melting point: 61˚C).

PREPARATION EXAMPLE 2

Preparation of t-butyl N-(4-bromo-3,5-bis(trifluoromethyl)phenyl)-N-(2-pyridinylmethyl)aminoacetate (compound No. 57)

(1) 500 mg of 4-bromo-3,5-bis(trifluoromethyl)aniline was dissolved in 3 mL of N,N-dimethylformamide, and 320 mg of t-butyl bromoacetate and then, 230 mg of potassium carbonate were added and reacted at about 100˚C overnight, and then the reaction solution was left to cool. To the reaction solution, water was added and stirred, and it was extracted with ethyl acetate. The crude product thereby obtained was purified by silica gel column chromatography (eluent: n-hexane/chloroform=2/3) to obtain 110 mg of t-butyl N-(4-bromo-3,5-bis(trifluoromethyl)phenyl)aminoacetate.

(2) 110 mg of the t-butyl N-(4-bromo-3,5-bis(trifluoromethyl)phenyl)aminoacetate was dissolved in 2 mL of N,N-dimethylformamide, and 80 mg of 2-bromomethylpyridine hydrogenbromide and then 25 mg of sodium hydride were added and reacted at about 60˚C for about 45 minutes, and then the reaction solution was left to cool. To the reaction solution, water was added and stirred, and it was extracted with ethyl acetate. The crude product thereby obtained was purified by silica gel column chromatography (eluent: n-hexane/ethyl acetate=3/7) to obtain 60 mg of the desired product (melting point: 77-78˚C)

PREPARATION EXAMPLE 3

Preparation of N-(4-bromo-3,5-bis(trifluoromethyl)phenyl)-N-(3-pyridinylmethyl)-2-pyridine methanamine (compound No. 36)

(1) 1.2 g of 4-bromo-3,5-bis(trifluoromethyl)aniline was dissolved in 10 mL of toluene, and 500 mg of 2-pyridine carboxyaldehyde and then a catalytic amount of pyridinium p-toluenesulfonate were added and reacted at about 100˚C overnight, and then the reaction solution was left to cool. To the reaction solution, 180 mg of sodium borohydride and 10 mL of ethanol were added and reacted at room temperature for about one hour. Then, water and ammonium chloride were added in proper amounts, followed by stirring. A crude product obtained by extraction with ethyl acetate was purified by silica gel column chromatography (eluent: n-hexane/ethyl acetate=2/3) to obtain 1.08 g of N-(4-bromo-3,5-bis(trifluoromethyl)phenyl)-2-pyridine methanamine (compound No. II-19).

(2) 100 mg of the N-(4-bromo-3,5-bis(trifluoromethyl)phenyl)-2-pyridine methanamine was dissolved in 1.5 mL of N,N-dimethylformamide, and 80 mg of 3-bromomethylpyridine hydrogenbromide and then 25 mg of sodium hydride were added and reacted at about 60˚C for about 30 minutes, and then, the reaction solution was left to cool. To the reaction solution, water was added and stirred, and then it was extracted with ethyl acetate. The crude product thereby obtained was purified by silica gel column chromatography (eluent: ethyl acetate/methanol=95/5) to obtain 90 mg of the desired product (oily product).

PREPARATION EXAMPLE 4

Preparation of N-(4-bromo-3,5-bis(trifluoromethyl)phenyl)-N-(thiophen-3-ylmethyl)-2-pyridine methanamine (compound No. 55)

(1) 300 mg of 4-bromo-3,5-bis(trifluoromethyl)aniline was dissolved in 2 mL of toluene, and 130 mg of 3-thiophene carboxyaldehyde and then a catalytic amount of pyridinium p-toluene sulfonate were added and reacted at about 100˚C overnight, and then the reaction solution was left to cool. To the reaction solution, 50 mg of sodium borohydride and 2 mL of ethanol were added and reacted at room temperature for about two hours. Then, water and ammonium

chloride were added in proper amounts, followed by stirring. A crude solution obtained by extraction with ethyl acetate was purified by silica gel column chromatography (eluent: n-hexane/ethyl acetate=2/1) to obtain 340 mg of N-(4-bromo-3,5-bis(trifluoromethyl)phenyl)-3-thiophene methanamine (melting point: 78-79˚C).

(2) 230 mg of N-(4-bromo-3,5-bis(trifluoromethyl)phenyl)-3-thiophene methanamine was dissolved in 3 mL of N,N-dimethylformamide, and 170 mg of 2-bromomethylpyridine hydrogenbromide and then 55 mg of sodium hydride were added and reacted at about 50˚C for about 30 minutes, and then, the reaction solution was left to cool. To the reaction solution, water was added and stirred, and then, it was extracted with ethyl acetate. The crude product thereby obtained was purified by silica gel column chromatography (eluent: n-hexane/ethyl acetate=2/3) to obtain 270 mg of the desired product (melting point: 58-59˚C).

PREPARATION EXAMPLE 5

Preparation of N-(4-cyano-3,5-bis(trifluoromethyl)phenyl)-N-(2-pyridinylmethyl)-2-pyridine methanamine (compound No. 65)

(1)1.0 g of 3,5-bis(trifluoromethyl)-4-bromoaniline was dissolved in 3 mL of hexamethylphosphoric acid triamide, and 730 mg of copper cyanide was added and reacted at 140˚C for 8 hours under irradiation with microwaves. The reaction solution was left to cool, and then, to the reaction solution, water and ethyl acetate were added, whereupon an insoluble solid content was removed by filtration through cerite. The organic layer was separated, and the aqueous layer was extracted twice with ethyl acetate. The organic layers were put together and washed with a saturated sodium chloride aqueous solution and then dried over anhydrous magnesium sulfate, and then, the solvent was distilled off under reduced pressure. The residue was purified by silica gel column chromatography (eluent: n-hexane/ethyl acetate=5/2) to obtain 550 mg of 3,5-bis(trifluoromethyl)-4-cyanoaniline as white solid.

(2) 280 mg of the 3,5-bis(trifluoromethyl)-4-cyanoaniline and 700 mg of (2-bromomethyl)pyridine hydrogenbromide were dissolved in 10 mL of dimethylsulfoxide, and 0.56 mL of a 10M sodium hydroxide aqueous solution was dropwise added, followed by a reaction at room temperature for one hour. To the reaction solution, a saturated ammonium chloride aqueous solution was added, and it was extracted twice with ethyl acetate, washed with a saturated sodium chloride aqueous solution and then dried over anhydrous magnesium sulfate, and then the solvent was distilled off under reduced pressure. The residue was purified by silica gel column chromatography (eluent: ethyl acetate/methanol=10/1) to obtain 290 mg of the desired product (melting point: 122˚C) as white solid.

Now, typical examples of the compound of the present invention will be given in Table 1. These compounds can be prepared by the above-described Preparation Examples or by the above-mentioned various processes for the production of the compound of the present invention. Further, typical examples of the compound represented by the above formula (II) will be given in Table 2. These compounds can be prepared based on the above-described production process [4], [5] or [6]. The compounds of the formula (II) are intermediates for the production of the compounds of the present invention, and in these compounds, those showing pesticidal activities, are included.

In Tables 1 and 2, No. represents the compound No., Me methyl, Et ethyl, and Bu(t) tertiary butyl. On the other hand, the temperature shown as the physical properties is the melting point, "oil" represents an oily substance. With respect to ones having the physical properties being "oil", $^1$H-NMR data was shown in Tables 3 and 4.

TABLE 1

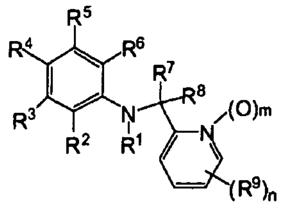

(I)

| No. | $R^1$ | $R^2$ | $R^3$ | $R^4$ | $R^5$ | $R^6$ | $R^7$ | $R^8$ | $(R^9)_n$ | m | Physical properties |
|---|---|---|---|---|---|---|---|---|---|---|---|
| 1 | 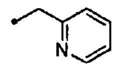 | H | H | Cl | $CF_3$ | H | H | H | n=0 | 0 | oil |
| 2 | 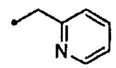 | H | H | CN | $CF_3$ | H | H | H | n=0 | 0 | oil |
| 3 | 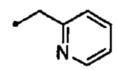 | H | H | $NO_2$ | $CF_3$ | H | H | H | n=0 | 0 | oil |
| 4 | 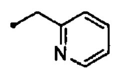 | H | H | $OCH_2CF_3$ | $CF_3$ | H | H | H | n=0 | 0 | oil |
| 5 | 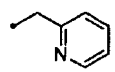 | H | Cl | $OCH_2CF_3$ | $CF_3$ | H | H | H | n=0 | 0 | oil |
| 6 | 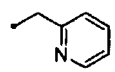 | OMe | H | H | $CF_3$ | H | H | H | n=0 | 0 | 80-81°C |
| 7 | 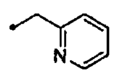 | $OCH_2CH_2OMe$ | H | H | $CF_3$ | H | H | H | n=0 | 0 | 47-48°C |
| 8 | 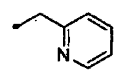 | H | Cl | Cl | $CF_3$ | H | H | H | n=0 | 0 | oil |
| 9 | 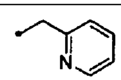 | H | Cl | Cl | $CF_3$ | H | H | H | n=0 | 1 | oil |
| 10 | 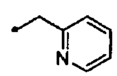 | Cl | H | Cl | $CF_3$ | H | H | H | n=0 | 0 | 97°C |

(continued)

| No. | $R^1$ | $R^2$ | $R^3$ | $R^4$ | $R^5$ | $R^6$ | $R^7$ | $R^8$ | $(R^9)_n$ | m | Physical properties |
|---|---|---|---|---|---|---|---|---|---|---|---|
| 11 | 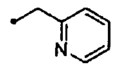 | F | H | Cl | $CF_3$ | H | H | H | n=0 | 0 | oil |
| 12 | 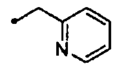 | H | H | Cl | $CF_3$ | Cl | H | H | n=0 | 0 | 69-72˚C |
| 13 | 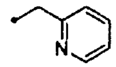 | F | H | Cl | $CF_3$ | Cl | H | H | n=0 | 0 | 74-76˚C |
| 14 | 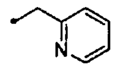 | H | Cl | SMe | $CF_3$ | H | H | H | n=0 | 0 | oil |
| 15 | 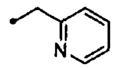 | H | $NO_2$ | H | $CF_3$ | H | H | H | n=0 | 0 | oil |
| 16 | 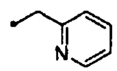 | H | Br | $NO_2$ | $CF_3$ | H | H | H | n=0 | 0 | oil |
| 17 | 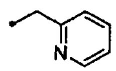 | H | Cl | $NO_2$ | $CF_3$ | H | H | H | n=0 | 0 | oil |
| 18 | 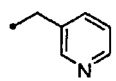 | H | Cl | $NO_2$ | $CF_3$ | H | H | H | n=0 | 0 | oil |
| 19 | 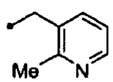 | H | Cl | $NO_2$ | $CF_3$ | H | H | H | n=0 | 0 | 123-125˚C |
| 20 | 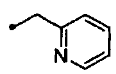 | H | Me | $NO_2$ | $CF_3$ | H | H | H | n=0 | 0 | oil |
| 21 | 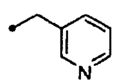 | H | Me | $NO_2$ | $CF_3$ | H | H | H | n=0 | 0 | oil |
| 22 | 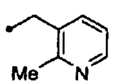 | H | Me | $NO_2$ | $CF_3$ | H | H | H | n=0 | 0 | 75-78˚C |
| 23 | 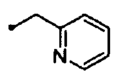 | H | OMe | $NO_2$ | $CF_3$ | H | H | H | n=0 | 0 | 113-114˚C |

(continued)

| No. | $R^1$ | $R^2$ | $R^3$ | $R^4$ | $R^5$ | $R^6$ | $R^7$ | $R^8$ | $(R^9)_n$ | m | Physical properties |
|---|---|---|---|---|---|---|---|---|---|---|---|
| 24 | 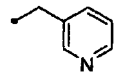 | H | OMe | $NO_2$ | $CF_3$ | H | H | H | n=0 | 0 | oil |
| 25 | 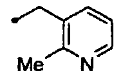 | H | OMe | $NO_2$ | $CF_3$ | H | H | H | n=0 | 0 | 149-150°C |
| 26 | 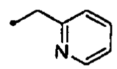 | H | $CF_3$ | H | $CF_3$ | H | H | H | n=0 | 0 | oil |
| 27 | 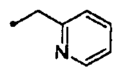 | H | $CF_3$ | Cl | $CF_3$ | H | H | H | n=0 | 0 | 61°C |
| 28 | 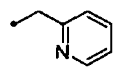 | H | $CF_3$ | Cl | $CF_3$ | H | Me | H | n=0 | 0 | oil |
| 29 | 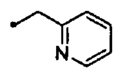 | H | $CF_3$ | Cl | $CF_3$ | H | H | H | n=0 | 1 | oil |
| 30 | 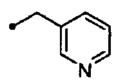 | H | $CF_3$ | Cl | $CF_3$ | H | H | H | n=0 | 0 | oil |
| 31 | 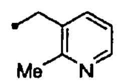 | H | $CF_3$ | Cl | $CF_3$ | H | H | H | n=0 | 0 | 92°C |
| 32 | $CH_2CH(OMe)_2$ | H | $CF_3$ | Cl | $CF_3$ | H | H | H | n=0 | 0 | oil |
| 33 | 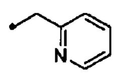 | H | $CF_3$ | Br | $CF_3$ | H | H | H | n=0 | 0 | 70°C |
| 34 | 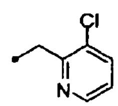 | H | $CF_3$ | Br | $CF_3$ | H | H | H | n=0 | 0 | oil |
| 35 | 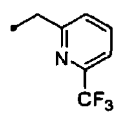 | H | $CF_3$ | Br | $CF_3$ | H | H | H | n=0 | 0 | oil |

(continued)

| No. | $R^1$ | $R^2$ | $R^3$ | $R^4$ | $R^5$ | $R^6$ | $R^7$ | $R^8$ | $(R^9)_n$ | m | Physical properties |
|---|---|---|---|---|---|---|---|---|---|---|---|
| 36 | 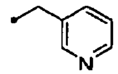 | H | $CF_3$ | Br | $CF_3$ | H | H | H | n=0 | 0 | oil |
| 37 | 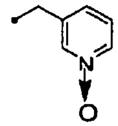 | H | $CF_3$ | Br | $CF_3$ | H | H | H | n=0 | 0 | oil |
| 38 | 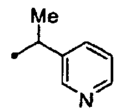 | H | $CF_3$ | Br | $CF_3$ | H | H | H | n=0 | 0 | oil |
| 39 | 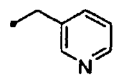 | H | $CF_3$ | Br | $CF_3$ | H | Me | H | n=0 | 0 | oil |
| 40 | 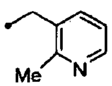 | H | $CF_3$ | Br | $CF_3$ | H | H | H | n=0 | 0 | 93-95˚C |
| 41 | 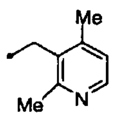 | H | $CF_3$ | Br | $CF_3$ | H | H | H | n=0 | 0 | oil |
| 42 | 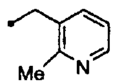 | H | $CF_3$ | Br | $CF_3$ | H | Me | H | n=0 | 0 | 100-101˚C |
| 43 | 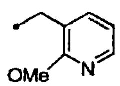 | H | $CF_3$ | Br | $CF_3$ | H | H | H | n=0 | 0 | oil |
| 44 | 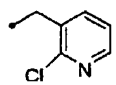 | H | $CF_3$ | Br | $CF_3$ | H | H | H | n=0 | 0 | oil |
| 45 | 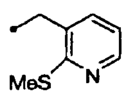 | H | $CF_3$ | Br | $CF_3$ | H | H | H | n=0 | 0 | 79-81˚C |

(continued)

| No. | $R^1$ | $R^2$ | $R^3$ | $R^4$ | $R^5$ | $R^6$ | $R^7$ | $R^8$ | $(R^9)_n$ | m | Physical properties |
|---|---|---|---|---|---|---|---|---|---|---|---|
| 46 | 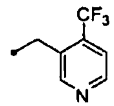 | H | $CF_3$ | Br | $CF_3$ | H | H | H | n=0 | 0 | 94-95˚C |
| 47 | 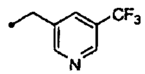 | H | $CF_3$ | Br | $CF_3$ | H | H | H | n=0 | 0 | 97-98˚C |
| 48 | 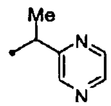 | H | $CF_3$ | Br | $CF_3$ | H | H | H | n=0 | 0 | oil |
| 49 | 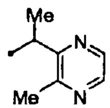 | H | $CF_3$ | Br | $CF_3$ | H | Me | H | n=0 | 0 | oil |
| 50 | 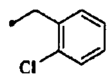 | H | $CF_3$ | Br | $CF_3$ | H | H | H | n=0 | 0 | oil I |
| 51 | 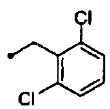 | H | $CF_3$ | Br | $CF_3$ | H | H | H | n=0 | 0 | 93-95˚C |
| 52 | 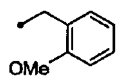 | H | $CF_3$ | Br | $CF_3$ | H | H | H | n=0 | 0 | oil |
| 53 | 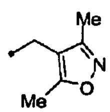 | H | $CF_3$ | Br | $CF_3$ | H | H | H | n=0 | 0 | 99-100˚C |
| 54 | 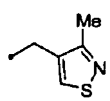 | H | $CF_3$ | Br | $CF_3$ | H | H | H | n=0 | 0 | 86-87˚C |
| 55 | 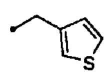 | H | $CF_3$ | Br | $CF_3$ | H | H | H | n=0 | 0 | 58-59˚C |

(continued)

| No. | $R^1$ | $R^2$ | $R^3$ | $R^4$ | $R^5$ | $R^6$ | $R^7$ | $R^8$ | $(R^9)_n$ | m | Physical properties |
|---|---|---|---|---|---|---|---|---|---|---|---|
| 56 | 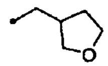 | H | $CF_3$ | Br | $CF_3$ | H | H | H | n=0 | 0 | oil |
| 57 | $CH_2COOBu$ (t) | H | $CF_3$ | Br | $CF_3$ | H | H | H | n=0 | 0 | 77-78˚C |
| 58 | 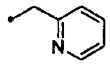 | H | $CF_3$ | H | $CF_3$ | Cl | H | H | n=0 | 0 | 88˚C |
| 59 | 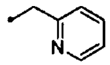 | H | Cl | $(CF_3)_2CF$ | Cl | H | H | H | n=0 | 0 | |
| 60 | 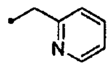 | H | H | H | $(CF_3)_2CF$ | Me | H | H | n=0 | 0 | |
| 61 | 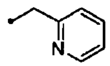 | H | Cl | $CF_3$ | Cl | H | H | H | n=0 | 0 | oil |
| 62 | 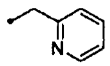 | H | $CF_3$ | SMe | $CF_3$ | H | H | H | n=0 | 0 | oil |
| 63 | $-CH_2CH(OMe)_2$ | H | $CF_3$ | Br | $CF_3$ | H | H | H | n=0 | 0 | oil |
| 64 | $-CH_2CH_2SMe$ | H | $CF_3$ | Br | $CF_3$ | H | H | H | n=0 | 0 | oil I |
| 65 | 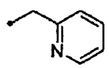 | H | $CF_3$ | CN | $CF_3$ | H | H | H | n=0 | 0 | 122˚C |
| 66 | $-CH_2CH(SEt)_2$ | H | $CF_3$ | Br | $CF_3$ | H | H | H | n=0 | 0 | 48˚C |
| 67 | 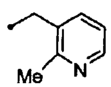 | H | $CF_3$ | CN | $CF_3$ | H | H | H | n=0 | 0 | 85-88˚C |
| 68 | 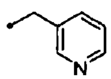 | H | $CF_3$ | CN | $CF_3$ | H | H | H | n=0 | 0 | oil |
| 69 | Hydrochloride of Compound No. 31 | | | | | | | | | | 165-169˚C |
| 70 | Hydrochloride of Compound No. 65 | | | | | | | | | | 162-164˚C |
| 71 | 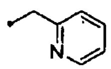 | H | H | H | $(CF_3)_2CF$ | H | H | H | n=0 | 0 | |

(continued)

| No. | $R^1$ | $R^2$ | $R^3$ | $R^4$ | $R^5$ | $R^6$ | $R^7$ | $R^8$ | $(R^9)_n$ | m | Physical properties |
|---|---|---|---|---|---|---|---|---|---|---|---|
| 72 | 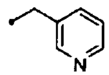 | H | H | H | $(CF_3)_2CF$ | H | H | H | n=0 | 0 | |
| 73 | 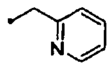 | H | $CF_3$ | $SCF_3$ | $CF_3$ | H | H | H | n=0 | 0 | |
| 74 | 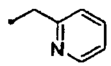 | H | $CF_3$ | SOMe | $CF_3$ | H | H | H | n=0 | 0 | oil |
| 75 | 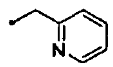 | H | $CF_3$ | $SO_2Me$ | $CF_3$ | H | H | H | n=0 | 0 | |
| 76 | 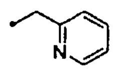 | H | $CF_3$ | Br | $CF_3$ | H | CN | H | n=0 | 0 | |
| 77 | 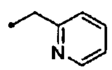 | H | $CF_3$ | CN | $CF_3$ | H | H | H | n=0 | 0 | |
| 78 | 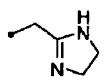 | H | $CF_3$ | Br | $CF_3$ | H | H | H | n=0 | 0 | |
| 79 | 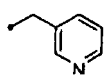 | H | $CF_3$ | $CF_3$ | $CF_3$ | H | H | H | n=0 | 0 | |
| 80 | 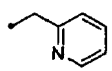 | H | $CF_3$ | $CF_3$ | $CF_3$ | H | H | H | n=0 | 0 | |
| 81 | 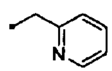 | H | $CF_3$ | CN | $CF_3$ | H | Me | H | n=0 | 0 | |
| 82 | 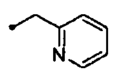 | H | H | $CHF_2$ | $CF_3$ | H | H | H | n=0 | 0 | |
| 83 | 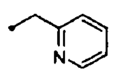 | H | H | 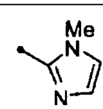 | $CF_3$ | H | H | H | n=0 | 0 | |

(continued)

| No. | R1 | R2 | R3 | R4 | R5 | R6 | R7 | R8 | $(R^9)_n$ | m | Physical properties |
|---|---|---|---|---|---|---|---|---|---|---|---|
| 84 | 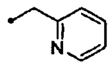 | H | H | 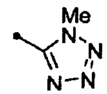 | $CF_3$ | H | H | H | n=0 | 0 | |
| 85 | 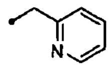 | H | Cl | Cl | Cl | H | H | H | n=0 | 0 | 101°C |
| 86 | 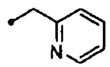 | H | CN | H | $CF_3$ | Cl | H | H | n=0 | 0 | 88°C |
| 87 | 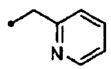 | H | CN | Cl | $CF_3$ | H | H | H | n=0 | 0 | oil |
| 88 | 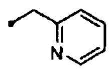 | H | $CF_3$ | Br | $CF_3$ | Br | H | H | n=0 | 0 | oil |
| 89 | 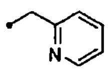 | H | $CF_3$ | Br | $CF_3$ | Me | H | H | n=0 | 0 | oil |

TABLE2

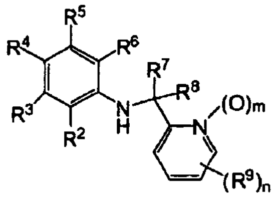

(II)

| No. | $R^2$ | $R^3$ | $R^4$ | $R^5$ | $R^6$ | $R^7$ | $R^8$ | $(R^9)_n$ | m | Physical properties |
|---|---|---|---|---|---|---|---|---|---|---|
| II-1 | H | H | Cl | $CF_3$ | H | H | H | n=0 | 0 | 60-62˚C |
| II-2 | H | H | Br | $CF_3$ | H | H | H | n=0 | 0 | 74-75˚C |
| II-3 | H | H | $NO_2$ | $CF_3$ | H | H | H | n=0 | 0 | 123-124˚C |
| II-4 | H | H | $OCH_2CF_3$ | $CF_3$ | H | H | H | n=0 | 0 | oil |
| II-5 | OH | H | H | $CF_3$ | H | H | H | n=0 | 0 | 163-166˚C |
| II-6 | OMe | H | H | $CF_3$ | H | H | H | n=0 | 0 | 91˚C |
| II-7 | OMe | H | H | $CF_3$ | Cl | H | H | n=0 | 0 | oil |
| II-8 | $OCH_2CH_2OMe$ | H | H | $CF_3$ | H | H | H | n=0 | 0 | 51˚C |
| II-9 | H | Cl | Cl | $CF_3$ | H | H | H | n=0 | 0 | 97˚C |
| II-10 | H | Cl | Cl | $CF_3$ | H | H | H | n=0 | 1 | 85-90˚C |
| II-11 | Cl | H | Cl | $CF_3$ | H | H | H | n=0 | 0 | 77-79˚C |
| II-12 | H | H | Cl | $CF_3$ | Cl | H | H | n=0 | 0 | 79-81˚C |
| II-13 | F | H | Cl | $CF_3$ | Cl | H | H | n=0 | 0 | 81-82˚C |
| II-14 | H | Me | $NO_2$ | $CF_3$ | H | H | H | n=0 | 0 | 79-81˚C |
| II-15 | H | OMe | $NO_2$ | $CF_3$ | H | H | H | n=0 | 0 | 117-118˚C |
| II-16 | H | $CF_3$ | H | $CF_3$ | Cl | H | H | n=0 | 0 | 127˚C |
| II-17 | H | $CF_3$ | Cl | $CF_3$ | Me | H | H | n=0 | 0 | 97-99˚C |
| II-18 | Me | $CF_3$ | Cl | $CF_3$ | Cl | H | H | n=0 | 0 | 50-51˚C |
| II-19 | H | $CF_3$ | Br | $CF_3$ | H | H | H | n=0 | 0 | 145-146˚2 |
| II-20 | H | $CF_3$ | Cl | $CF_3$ | H | H | H | n=0 | 0 | 126-127˚C |
| II-21 | H | $CF_3$ | Br | $CF_3$ | H | Me | H | n=0 | 0 | oil |

TABLE 3

| No. | $^1H$-NMR δppm ( Solvent : $CDCl_3$ /400MHz) |
|---|---|
| 1 | 4.83 (4H, s), 6.76 (1H, dd. J = 8.8, 2.8Hz), 7.03 (1H, d, J = 3.6Hz) 7. 19-7.24(5H, m), 7.68-7.71(2H, m), 8.60-8.62 (2H, m) |
| 2 | 4.90(4H, s), 6.86(1H, dd, J=8.8, 2.8Hz), 7.09(1H, d, J=2.4Hz) 7. 18-7.27 (4H, m), 7.53(1H, d, J = 8. 8Hz), 7. 65-7. 72 (2H, m), 8. 6 2(2H, dd, J = 4. 8, 1. 2Hz) |
| 3 | 4.93(4H, s), 6.84(1H, dd, J = 9. 2, 3.2Hz), 7.17(1H, d, J = 2. 4Hz) 7. 20-7. 26 (4H, m), 7. 66-7. 70 (2H, m), 7.95(1H, d, J=9.2Hz). 8.6 2(2H, dd, J = 4. 8, 1.2Hz) |
| 4 | 4.27(2H, q, J = 7.6Hz), 4.80(4H, s) , 6.80(1H, dd, J = 8. 8, 3. 2Hz ), 6.86(1H, d, J = 8. 8Hz), 6. 97(1H, d, J = 2. 8Hz), 7. 18-7.26 (4H, m), 7. 62-7. 67 (2H, m), 8.59-8.61 (2H, m) |

(continued)

| No. | $^1$H-NMR δppm ( Solvent : $CDCl_3$ /400MHz) |
|---|---|
| 5 | 4.28(2H, q, J = 8. 0 Hz), 4.80(4H, s), 6.87(1H, d, J = 2. 8Hz), 6. 9 0 (1H, d, J = 2. 8Hz), 7.20-7.23(4H, m), 7.65-7.69(2H, m), 8.61-8.6 2(2H, m) |
| 8 | 4.81 (4H, s), 6.98(1H, d, J = 3. 6Hz), 7.00(1H, d, J = 3. 2Hz), 7. 1 9-7. 23 (4H, m), 7. 64-7. 68 (2H, m), 8.60-8.62 (2H, m) |
| 9 | (Solvent: DMSO-$d_6$): 4.89(2H, s), 4.91(2H, s), 6.98(1H, d, J = 2. 8Hz), 7.11(1H, d, J = 2. 8Hz), 7.27-7.30(2H, m), 7.35-7.49(2H, m), 7. 74-7. 81 (2H, m), 8.34 (1H, d= 6.4Hz), 8.54 (1H, d = 4. 4Hz) |
| 11 | 4.63(4H, s). 7. 14-7. 19 (3H, m), 7. 29 (1H, d, J = 8. 8Hz), 7.35 (2H, d J = 8.0Hz), 7,64 (2H, td, J = 8. 0, 2. 0Hz), 8.54-8. 56 (2H, m) |
| 14 | 2.29(3H, s), 4.83(4H, s), 7. 00-7. 23 (6H, m), 7. 65-7. 69 (2H, m), 8.6 1 (2H. J = 4. 8Hz) |
| 15 | 4.91(4H, s), 7.22-7.26(5H, m), 7.67(2H, td, J=7.6, 1.6Hz), 7. 6 9-7. 77 (2H, m), 8. 62-8. 64 (2H, m) |
| 16 | 4.86(4H, s), 6.99(1H, d, J = 2. 4Hz), 7.11(1H, d, J = 3.2Hz), 7. 1 9-7.25(4H, m), 7.66-7.71 (2H, m), 8.62(2H, dd, J=4.0, 1.2Hz) |
| 17 | 4.86(4H, s), 6.93-6.95 (2H, d, m), 6.95(1H, s), 6.70(1H, s), 7. 19-7.25(2H, m), 7.67-7.71(2H,m), 8.61-8.63 (2H, m) |
| 18 | 4.78(2H, s), 4.83(2H, s), 6.91(2H, s), 7. 17 (1H, d, J=8.0Hz), 7. 25-7. 38 (2H, m), 7. 53-7. 55 (1H, m), 7. 66-7. 72 (1H, m), 8.54 (1H, d, J = 2. 0Hz), 8. 56-8. 62 (2H, m) |
| 20 | 2.23(3H, s), 4.87(2H, s), 6.68(1H, d, J = 2. 8Hz), 6.87(1H, d, J = 2. 4Hz), 7. 19-7. 25 (4H, m), 7. 65-7. 70 (2H, m), 8.61-8. 63 (2H, m) |
| 21 | 2.25(3H, s), 4.78(2H, s), 4.82(2H, s), 6.67(1H, d, J = 2.4Hz), 6. 84(1H, d, J=3.2Hz), 7.16(1H, d, J = 7. 6Hz), 7.23-7.32(2H, m), 7 53-7.56 (1H, m), 7. 63-7. 70 (1H, m), 8.54(1H, d, J =1.6Hz), 8. 56-8 62(2H, m) |
| 24 | 3.70(3H, s), 4.79(2H, s), 4.85(2H, s), 6.43(1H, d, J=2.4Hz), 6. 55(1H, d, J = 2.4Hz), 7.18 (1H, d, J = 7.6Hz), 7. 23-7. 32 (2H, m), 7 .55-7.57(1H, m), 7. 67-7.71(1H, m), 8. 57-8. 62 (3H, m) |
| 26 | 4.88(4H, s), 7.11(2H, s), 7.16(1H, s), 7.20-7.23(4H, m), 7.64-7.6 8(2H, m), 8.62(1H, d, J =5. 6, 2.8Hz) |
| 28 | 1.73(3H, d, J = 6.8Hz), 4.69(1H, d, J = 18.0Hz), 4.86(1H, d, J = 18.0Hz), 5.28(1H, q, J = 6.8Hz), 7. 10-7.26 (6H, m), 7.57-7.66 (2H, m), 8.561H, d, J = 4.8Hz), 8.59(1H, d, J = 4. 4Hz) |
| 29 | (Solvent : DMSO-$d_6$): 4.94(2H. s), 4.99(2H, d), 7.22(2H, s). 7. 27-7.46(4H, m), 7. 74-7. 84 (2H, m), 8.36(1H, d= 6.4Hz), 8.54(1H, d = 4 .8Hz) |
| 30 | 4.77(2H, s), 4.81(2H, s), 7.13-7.31(5H, m), 7.54-7, 57(1H, m), 7.6 5-7.70(1H, m), 8.55-8.62 (3H, m) |
| 32 | 3.28(6H, s), 3.56(2H, d, J=5.2Hz), 4.53(1H, t, J=5.2Hz), 4. 59 (2H, s) , 7.14-7.18(1H, m), 7.39-7.54 (1H, m), 7.59(2H,s), 7.61-7. 66(1H, m), 8. 52 (1H, d, J 4.8Hz) |
| 34 | 2.65(3H, s), 4.66(2H, s), 4.76(2H, s), 6. 96-7. 00 (1H, m), 7.05(2H, s), 7.12-7.31(3H, m), 7.65-7.70(1H, m), 8.41-8.43(1H, m), 8.61(1 H, d, J = 4.0Hz) |
| 35 | 4.86(2H, s), 4.92(2H, s), 7. 22-7. 26 (2H, m), 7.25(2H, s), 7.44(1H, d, J = 8.4Hz), 7.61-7.70 (2H, m), 7.86 (1H, t, J = 8.0Hz), 8.60-8. 62 (1H, m) |
| 36 | 4.77(2H, s), 4.81(2H, s), 7.17 (1H, d, J=8.0Hz), 7.20(2H, s), 7. 22-7. 29 (2H, m), 7.55 (1H, d, J = 8. 0Hz), 7. 65-7. 70 (1H, m), 8. 02-8. 61 (3H, m) |
| 37 | 4.75 (2H, s), 4.78 (2H, s), 7.10-7.32(6H, m), 7.68-7.72(1H, m), 8. 1 6-8.19(2H, m), 8.58-8.61 (1H, m) |
| 38 | 1.72 (3H, d, J=7.2Hz), 4.56 (1H, d, J =17.6Hz), 4.65(1H, d, J = 17.6Hz), 5.32(1H, q, J = 7. 2Hz), 7.06(1H, d, J = 8. 0Hz), 7. 15-7. 8(1H, m), 7.23(2H, s), 7.26-7.29 (1H, m), 7.57-7.64 (2H, m), 8. 53-8 55(2H, d, m), 8.63 (1H, d, J = 2.0Hz) |
| 39 | 1.72 (3H,d, J = 7.2Hz), 4.62(1H, d, J = 17.6Hz), 4.77(1H, d, J = 17.6Hz), 5.27 (1H, q, J = 7. 2Hz), 7. 18-7.32 (5H, m), 7.42-7.47 (1H, m), 7. 63-7. 68(1H, m), 8.45(1H, d, J = 1.2Hz), 8.48-8.50 (1H, m), 8 58-8. 59(1H, m) |
| 41 | 2.24 (3H, s), 2.47(3H, s), 4.40(2H, s), 4.73(2H, s), 6.89(1H, d, J = 7.6Hz), 6.96(1H, d, J = 4.8Hz), 7. 12-7. 15 (1H, m), 7.38(2H, s), 7.54-7.58 (1H, m), 8.31 (1H, d, J = 4.8Hz), 8.50(1H, d, J = 4.8Hz) |

(continued)

| No. | $^1$H-NMR δppm ( Solvent : $CDCl_3$ /400MHz) |
|---|---|
| 43 | 3.99(3H, s), 4.69(2H, s), 4.77(2H, s), 6.86(1H, dd, J=7.2, 4. 8H z) 7.13(1H, d, J=8.0Hz), 7.17(2H, s), 7.21-7.24 (1H,m), 7.36(1H, dd, J = 7.2, 2.0Hz) 7.63-7.68 (1H, m), 8.12-8.13 (1H, m), 8.61 (1H, d, J = 4.8Hz) |
| 44 | 4.77(2H, s), 4.82(2H, s). 7.12(2H, s), 7. 17-7. 26 (3H, m), 7.49-7.5 2(1H, m), 7.67-7.72 (1H, m), 8.37(1H, dd, J =5.2, 2.0Hz) 8.61(1H, d, J = 4.4Hz) |
| 48 | 1.76(3H, d, J = 6.8Hz), 4.68(1H, d, J = 17.2Hz), 4.82(1H, d, J = 18.0Hz), 5.36 (1H, q, J = 6.8Hz), 7.08(1H, d, J = 8.0Hz), 7.15-7.1 8(1H, m), 7.25(2H, s), 7. 57-7. 62 (1H, m), 8.48(1H, d, J=2.4Hz), 8. 54-8. 56 (2H, m) 8.62 (1H, d, J = 1.6Hz) |
| 49 | 1.58(3H,s), 1.71(3H, d, J = 7.2Hz), 4.64(1H, d, J = 17.6Hz), 4.69 (1H, d, J = 17.6Hz), 5.39(1H, q, J = 7.2Hz), 6.78(1H, d, J = 8.0H z), 7.07-7.10(1H, m), 7.31(2H, s), 7.45-7.50(1H, m), 8.29(1H, d, J = 2.4Hz), 8.34(1H, d, J = 2.4Hz) 8.45-8.47(1H, m) |
| 50 | 4.78(2H, s), 4.83(2H, s), 7.13(2H, s). 7.15-7.29(5H, m), 7.43-7.4 5(1H, m), 7.66-7.70(1H, m), 8.61(1H, d, J =4. 8Hz) |
| 52 | 3.85(3H, s), 4.73(2H, s), 4.78(2H, s), 6. 89-6. 97 (2H, m), 7.10-7.1 2(2H, m), 7.19-7.3(4H, m), 7.61-7.65(1H, td, J=7.6, 2.0Hz), 8. 60(1H, d, J = 4.8Hz) |
| 56 | 1.24-1.70 (1H. m), 2.06-2.13(1H. m), 2.69-2.74(1H, m), 3. 50-3. 62 (3 H, m), 3.75-3.81 (2H, m), 3. 93-3. 98 (1H, m), 4.72(2H, s), 7.06(1H, d, J = 8.0Hz), 7.16 (2H, s), 7.19-7.24(1H, m), 7.61-7.67(1H, m), 8 59(1H, d, J = 4.8Hz) |
| 61 | 4.80(4H. m), 6.73(2H, s), 7.17-7.25 (4H, m), 7.65-7.70 (2H, m), 8.6 2(2H, dd, J = 4.8, 1.2Hz) |
| 62 | 2.82 (3H, s), 4.86(4H, s), 7.20(2H, s), 7. 21-7. 26 (4H, m), 7.65(2H, td, J = 8.0, 5.2Hz), 8.62(2H, d, J 5.2Hz) |
| 63 | 3.43(6H, s), 3.67(2H, d, J = 4.8Hz), 4.58(1H, t, J = 4.8Hz), 4. 77 (2H, s), 7.11(1H, d, J = 8.0Hz), 7.21(2H, s), 7. 19-7.22 (1H, m), 7 62 (1H, td, J = 8.0, 5.2Hz), 8.60(1H, d. J = 5. 2Hz) |
| 64 | 2.19(3H, s), 2.78(2H, t, J = 7.6Hz), 3.77(2H, t, J = 7.2Hz), 4.72 (2H, s), 7.14 (1H, d, J = 8.0Hz), 7.15 (2H, s), 7. 19-7.23 (1H, m), 7 65(1H, td, J = 8.0, 4.0Hz), 8.60(H, d, J = 4.0Hz) |
| 68 | 4.84 (2H, s), 4.88(2H, s), 7.19(2H, s), 7. 26-7. 33 (3H, m), 7.55 (1H, d, J = 8.0Hz), 7.64(1H, td, J = 8.0, 4.0Hz), 8.54(1H, s), 8. 57-8 60(2H, m) |
| 74 | 2.93 (3H, s), 4.92 (4H, s). 7. 23-7. 27 (4H, m). 7.32 (2H, s), 7.68 (dt 2H, *J* = 5.2, 8.0Hz). 8.62(2H, dd, *J* = 1.2, 4. 8Hz) |
| 87 | 4.84(4H, s), 7. 12-7. 33 (6H, m), 7.66(dt, 2H, *J* = 4.8, 8.0Hz), 8.6 2 (2H, d, *J* = 4.8Hz) |
| 88 | 4.40(4H, s), 7.16-7.20(2H, m), 7.27(2H, d. *J* =8.0Hz), 7.55(1H, s), 7.66(2H, dt, *J* = 5.2, 8.0Hz), 8.57(2H, d, *J* = 4.4Hz) |
| 89 | 2.64(3H, s), 9.39(4H, s), 7. 18-7.25 (2H, m) , 7. 28 (2H, d, *J* = 8.0H z), 7.42(1H, s), 7.63(2H, dt, *J* = 5.2, 8.0Hz), 8.52(2H, d, J = 4 8Hz) |

TABLE4

| No. | $^1$H-NMR δppm (Solvent : $CDCl_3$ /400MHz) |
|---|---|
| II-4 | 4.30(2H, q, J = 8.4Hz), 4.43(2H, s), 6.7(1H, dd, J = 8.8, 3.2Hz), 6.90-6.92 (2H, m), 7.20-7.23 (1H, m), 7.31 (1H, d, *J* = 7.6Hz), 7.6 5-7.70 (1H, m), 8.59-8.61 (1H, m) |
| II-7 | 3.86 (3H, s), 4.65(2H, s), 6.75(1H, d, J = 8.8Hz), 7.16-7.20(2H, m ), 7. 26-7. 30(1H, m), 7. 62-7.66(1H, m), 8. 58-8. 60(1H. m) |
| II-21 | 1.56(3H, d, J= 6.4Hz), 4.61-4.68(1H, m). 5.31(1H, d, J = 6.8Hz), 7.12(2H, s), 7.20-7.23 (1H, m), 7.28-7.30 (1H, m), 7.65-7.71(1H, m ), 8. 58-8. 59 (1H, m) |

Now, Test Examples will be described.

TEST EXAMPLE 1

Test on controlling effects against green peach aphid (Myzus persicae)

A Japanese radish leaf was inserted in a test tube in which water was put, and about 20 first instar nymphs of green peach aphid were released on the leaf. On the next day, the number of nymphs parasitic on the leaf was counted, and then the leaf was dipped for about 10 seconds in an insecticidal solution prepared to bring the concentration of the compound of the present invention to 800 ppm, dried in air and left in a constant temperature chamber at 25°C with lightening. Survived nymphs were counted 5 days after the treatment, and the mortality was calculated by the following equation. The insects that dropped from the leaf or were moribund were included in the number of dead. The test was carried out with respect to the above-mentioned compound Nos. 8, 14, 16-22, 24, 27-31, 33-38, 40, 41, 43, 44, 45, 48, 49, 53, 56, 61-70, 87 and 11-5, whereby all compounds showed a mortality of at least 90%.

$$\text{Mortality (\%)}=(1-(\text{number of survived insects}/\text{number of treated insects}))\times 100$$

TEST EXAMPLE 2

Test on controlling effects against common cutworm (Spodoptera litura)

A cabbage leaf disk was dipped for about 10 seconds in an insecticidal solution prepared to bring the concentration of the compound of the present invention to 800 ppm and dried in air. In a Petri dish having a diameter of 9 cm, a wet filter paper was placed, and the dried cabbage leaf fragment was placed thereon. Then, 10 second-third instar larvae of common cutworm were released thereon and after putting a cover on the Petri dish, left in a constant temperature chamber at 25°C with lightening. On the fifth day after the release, dead larvae were counted, and the mortality was calculated by the following equation. Moribund larvae were included in the number of dead. The test was carried out with respect to the above-mentioned Compound Nos.3, 8, 9, 16-25, 27-31, 33-38, 40, 41, 43-46, 48, 49, 50, 53, 56, 61, 62, 64-70 and 87, whereby all compounds showed a mortality of at least 90%.

$$\text{Mortality (\%)} = (\text{Number of dead larvae}/\text{number of released larvae}) \times 100$$

TEST EXAMPLE 3

Test on adults of two-spotted spider mite (Tetranychus urticae)

An insecticidal solution was prepared to bring the concentration of the compound of the present invention to 800 ppm. A kidney bean having only one primordial leaf left, was transplanted to a pot (diameter: 8 cm, height: 7 cm), and 20 adults of two-spotted spider mite were released thereon. Together with the kidney bean leaf, they were dipped in the above insecticidal solution, dried in air and then left in a constant temperature chamber at 25°C with lightening. On the second or third day after the treatment, dead adults were counted, and the mortality of adults was calculated by the following equation. Adults that dropped from the leaf or were moribund were included in the number of dead. The test was carried out with respect to the above-mentioned Compound No. 1, 2, 3, 8, 9, 16-22, 24, 26-31, 33-41, 43, 44, 45, 48, 49, 50, 52, 53, 56, 61-70, II-1 and II-17 whereby all compounds showed a mortality of adults of at least 90%.

$$\text{Mortality of adults (\%)} = (\text{Number of dead two-spotted spider mites}/\text{Number of treated two-spotted spider mites}) \times 100$$

TEST EXAMPLE 4

Test on controlling effects against Haemaphysalis longicornis

On an inner surface of a Petri dish having a diameter of 9 cm, 1 ml of a solution of the compound of the present invention in acetone (concentration: 10 μg/ml) is dropped by a micro pipette. After the inner surface of the Petri dish is dried, 60 to 180 Larval ticks are put, and the Petri dish is covered with a polyethylene sheet and sealed by a rubber band. The number of ticks knocked down after contact with the compound is counted, whereby most of the compounds

of the present invention will knock down Haemaphysalis longicornis.

TEST EXAMPLE 5

Test on controlling effects against Haemaphysalis longicornis employing a dog

50 Young mites of Haemaphysalis longicornis are released on the auricle of a dog (Beagle, 8 month old) and artificially parasitized. On the second day after being parasitized, the number of parasitic ticks is counted, and then a formulated compound of the present invention is spotted on the back of neck in an amount of 10 mg/kg.
After administration of the drug, observation is continued up to the fifth day, whereby the number of parasitic ticks, the number of dropped ticks and the life or death of the dropped ticks are determined. The dog is independently taken care in a separate cage, permitted to freely drink tap water and fed with a predetermined amount of a dog food once a day. As a result, the compounds of the present invention are effective to kill or drop the parasitic Haemaphysalis longicornis.

TEST EXAMPLE 6

Test on controlling effects against cat flea (Ctenocephalides felis)employing a dog

100 Non-blood sucked adults of cat flea within three days after adult emergence, are released on the dorsal fur of a dog (Beagle, 8 month old) and artificially parasitized, and on the back of neck, a formulated compound of the present invention is spotted on at a dose of 10 mg/kg. On the third day after administration of the compound, the cat flea is recovered by means of a flea catching comb, and the parasitized number is counted. The dog is individually taken care in a separate cage, permitted to freely drink tap water and fed with a predetermined amount of a dog food once a day. As a result, the compound of the present invention is effective to control the parasitizing of cat flea.

TEST EXAMPLE 7

Test on controlling effects against Haemaphysalis longicornis

On an inner surface of a Petri dish having a diameter of 9 cm, 1 ml of a solution of the compound No. 40 in acetone (concentration: 10 μg/ml, 1 μg/ml or 0.1 μg/ml) was dropped by a micro pipette. After the inner surface of the Petri dish was dried, 100 Larval ticks (Haemaphysalis longicornis) were put, and the Petri dish was covered with a polyethylene sheet and sealed by a rubber band. Thereafter, the Petri dish was left to stand still at a constant temperature of 25°C under a relative humidity of 100% under a constant dark condition except for the time of observation. Observation was carried out every time upon expiration of a certain time (i.e. after 5, 10, 20, 30, 60, 120, 180 and 240 minutes), and the number of ticks knocked down after contact with the compound was counted. As a control group, Larval ticks were put in a Petri dish having 1 ml of acetone dropwise added and observed in the same manner. The above operation was repeated twice.
From the number of knocked down ticks in each observation, the survival rate was obtained, and a corrected mortality was calculated by the following Abbott correction formula. Then, probit-time linear line was drawn, and the median knock down time (KT50 value) was obtained. The test results are shown in Table 5.

Corrected mortality (%) = [(Survival rate in control group - Survival rate in treated group)/Survival rate in control group] × 100

TABLE 5

| KT50 value (min) of compound No. 40 | | | |
|---|---|---|---|
| Concentration | First time | Second time | Average |
| 10 μg/ml | 40 | 40 | 40 |
| 1 μg/ml | 50 | 50 | 50 |
| 0.1 μg/ml | 40 | 35 | 38 |

TEST EXAMPLE 8

Test on controlling effects against cat flea (Ctenocephalides felis)

About 25 adults of cat flea to be tested are put in a vial containing filter paper treated with the compound of the present invention. Upon expiration of 24, 48 and 72 hours after putting the adults, survived and dead fleas are counted. The compound of the present invention is effective to kill the majority of cat fleas.

Now, Formulation Examples are described below.

FORMULATION EXAMPLE 1

| | | |
|---|---|---|
| (1) | Compound of the present invention | 20 parts by weight |
| (2) | Clay | 70 parts by weight |
| (3) | White carbon | 5 parts by weight |
| (4) | Sodium polycarboxylate | 3 parts by weight |
| (5) | Sodium alkylnaphthalene sulfonate | 2 parts by weight |

The above components are uniformly mixed to obtain a wettable powder.

FORMULATION EXAMPLE 2

| | | |
|---|---|---|
| (1) | Compound of the present invention | 5 parts by weight |
| (2) | Talc | 60 parts by weight |
| (3) | Calcium carbonate | 34.5 parts by weight |
| (4) | Liquid paraffin | 0.5 part by weight |

The above components are uniformly mixed to obtain a dust.

FORMULATION EXAMPLE 3

| | | |
|---|---|---|
| (1) | Compound of the present invention | 20 parts by weight |
| (2) | N, N-dimethylacetamide | 20 parts by weight |
| (3) | Polyoxyethylene tristyryl phenyl ether | 10 parts by weight |
| (4) | Calcium dodecylbenzene sulfonate | 2 parts by weight |
| (5) | Xylene | 48 parts by weight |

The above components are uniformly mixed and dissolved to obtain an emulsifiable concentrate.

FORMULATION EXAMPLE 4

| | | |
|---|---|---|
| (1) | Clay | 68 parts by weight |
| (2) | Sodium lignin sulfonate | 2 parts by weight |
| (3) | Polyoxyethylene alkylaryl sulfate | 5 parts by weight |
| (4) | White carbon | 25 parts by weight |

The mixture of the above components is mixed with compound of the present invention in a weight ratio of 4:

1 to obtain a wettable powder.

FORMULATION EXAMPLE 5

| | | |
|---|---|---|
| (1) | Compound of the present invention | 50 parts by weight |
| (2) | Sodium alkylnaphthalene sulfonate condensation product of formaldehyde | 2 parts by weight |
| (3) | Silicone oil | 0.2 part by weight |
| (4) | Water | 47.8 parts by weight |

The above components are uniformly mixed and pulverized to obtain a base liquid, and

| | | |
|---|---|---|
| (5) | Sodium polycarboxylate | 5 parts by weight |
| (6) | Anhydrous sodium sulfate | 42.8 parts by weight |

are added, and the mixture is uniformly mixed, granulated and dried to obtain water-dispersible granules.

FORMULATION EXAMPLE 6

| | | |
|---|---|---|
| (1) | Compound of the present invention | 5 parts by weight |
| (2) | Polyoxyethylene octyl phenyl ether | 1 part by weight |
| (3) | Polyoxyethylene alkyl ether phosphoric acid ester | 0.1 part by weight |
| (4) | Granular calcium carbonate | 93.9 parts by weight |

The above components (1) to (3) are preliminarily uniformly mixed and diluted with a proper amount of acetone, and then the mixture is sprayed onto the component (4), and acetone is removed to obtain granules.

FORMULATION EXAMPLE 7

| | | |
|---|---|---|
| (1) | Compound of the present invention | 2.5 parts by weight |
| (2) | N,N-dimethylacetamide | 2.5 parts by weight |
| (3) | Soybean oil | 95.0 parts by weight |

The above components are uniformly mixed and dissolved to obtain an ultra low volume formulation.

FORMULATION EXAMPLE 8

| | | |
|---|---|---|
| (1) | Compound of the present invention | 40 parts by weight |
| (2) | Potassium polyoxyethylene styryl phenyl ether phosphate | 4 parts by weight |
| (3) | Silicone oil | 0.2 part by weight |
| (4) | Xanthan gum | 0.1 part by weight |
| (5) | Ethylene glycol | 5 parts by weight |
| (6) | Water | 50.7 parts by weight |

The above components are uniformly mixed and pulverized to obtain a water-based suspension concentrate.

FORMULATION EXAMPLE 9

| | | |
|---|---|---|
| (1) | Compound of the present invention | 10 parts by weight |
| (2) | Diethylene glycol monoethyl ether | 80 parts by weight |
| (3) | Polyoxyethylene alkyl ether | 10 parts by weight |

The above components are uniformly mixed to obtain a soluble concentrate.

INDUSTRIAL APPLICABILITY

The novel N-phenyl-methanamine derivative of the present invention has a very high pesticidal effect against pests at a low dose, and it also has safety to crop plants, the natural enemy to pests, or mammals.

The entire disclosure of Japanese Patent Application No. 2007-165618 filed on June 22, 2007 including specification, claims and summary is incorporated herein by reference in its entirety.

## Claims

**1.** An N-phenyl-methanamine derivative represented by the formula (I) or its salt:

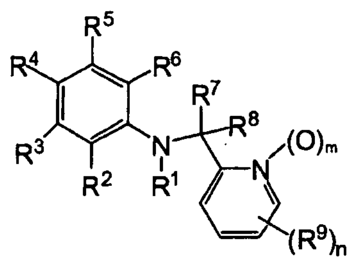

(I)

wherein $R^1$ is alkyl which may be substituted by $R^b$, alkenyl which may be substituted by $R^b$, alkynyl which may be substituted by $R^b$, aryl which may be substituted by $R^d$, or a heterocyclic group which may be substituted by $R^d$; each of $R^2$, $R^3$, $R^4$ and $R^6$ which are independent of one another, is hydrogen, halogen, cyano, isonitrile, nitro, alkyl which may be substituted by $R^b$, alkenyl which may be substituted by $R^b$, alkynyl which may be substituted by $R^b$, cycloalkyl, aryl, a heterocyclic group which may be substituted by alkyl, $NR^aR^c$, $OR^a$, $S(O)_pR^a$, $COR^a$, $COOR^a$ or $CONR^aR^c$; $R^5$ is haloalkyl or halogen; each of $R^7$ and $R^8$ which are independent of each other, is hydrogen, cyano, alkyl, alkenyl, alkynyl, haloalkyl or cycloalkyl, or $R^7$ and $R^8$ may together form $C_{3-6}$ cycloalkyl which may be substituted by halogen; $R^9$ is alkyl, cycloalkyl, alkoxyalkyl, alkoxyalkoxyalkyl, hydroxyalkyl, halogen, haloalkyl, cyano, nitro, aryl which may be substituted by halogen, a heterocyclic group which may be substituted by halogen, heterocyclic oxy which may be substituted by halogen, $CONR^aR^c$, $COR^a$, $NR^aR^c$, $COOR^a$ or $OR^a$; each of $R^a$ and $R^c$ which are independent of each other, is hydrogen, alkyl, haloalkyl, heterocyclic alkyl, alkoxyalkyl, hydroxyalkyl, cycloalkyl, aryl which may be substituted by $R^d$, or a heterocyclic group which may be substituted by $R^d$; $R^b$ is halogen, aryl which may be substituted by $R^d$, a heterocyclic group which may be substituted by $R^d$, an N-oxide of a nitrogen-containing heterocyclic group which may be substituted by $R^d$, heterocyclic oxy which may be substituted by $R^d$, heterocyclic thio which may be substituted by $R^d$, cyano, $NR^aR^c$, $NHCOOR^a$, $OR^a$, $COR^a$, $COOR^a$ or $S(O)_pR^a$; $R^d$ is alkyl, cycloalkyl, alkoxyalkyl, alkoxyalkoxyalkyl, hydroxyalkyl, halogen, haloalkyl, cyano, nitro, aryl, a heterocyclic group which may be substituted by halogen, heterocyclic oxy which may be substituted by halogen, $COR^a$, amino, monoalkylamino, dialkylamino, $COOR^a$, $OR^a$ or $SR^a$; m is an integer of from 0 to 1; n is an integer of from 0 to 4, p is an integer of from 0 to 2, in the $NR^aR^c$ moiety in each of the above substituents, $R^a$ and $R^c$ may together form a 5- or 6-membered heterocyclic ring together with the nitrogen atom to which they are bonded, provided that 2-[bis (2-pyridinylmethyl)amino]-4-chlorophenol and 2-methoxy-5-chlorophenyl-bis(2-pyridylmethyl)amine are excluded.

**2.** The N-phenyl-methanamine derivative or its salt according to Claim 1, wherein $R^1$ is alkyl which may be substituted by $R^b$; each of $R^2$, $R^3$, $R^4$ and $R^6$ which are independent of one another, is hydrogen, halogen, cyano, nitro, alkyl which may be substituted by $R^b$, $OR^a$ or $S(O)_pR^a$; each of $R^7$ and $R^8$ which are independent of each other, is

hydrogen or alkyl; m is 0, n is 0, and p is 0.

**3.** The N-phenyl-methanamine derivative or its salt according to Claim 1, wherein at least one of $R^2$, $R^3$, $R^4$ and $R^6$ is halogen or cyano.

**4.** A method for producing an N-phenyl-methanamine derivative represented by the formula (I) or its salt:

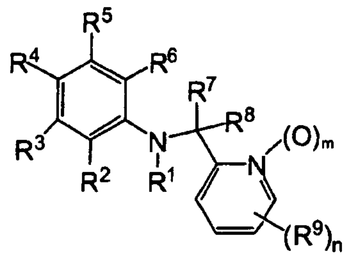

(I)

wherein $R^1$ is alkyl which may be substituted by $R^b$, alkenyl which may be substituted by $R^b$, alkynyl which may be substituted by $R^b$, aryl which may be substituted by $R^d$, or a heterocyclic group which may be substituted by $R^d$; each of $R^2$, $R^3$, $R^4$ and $R^6$ which are independent of one another, is hydrogen, halogen, cyano, isonitrile, nitro, alkyl which may be substituted by $R^b$, alkenyl which may be substituted by $R^b$, alkynyl which may be substituted by $R^b$, cycloalkyl, aryl, a heterocyclic group which may be substituted by alkyl, $NR^aR^c$, $OR^a$, $S(O)_pR^a$, $COR^a$, $COOR^a$ or $CONR^aR^c$; $R^5$ is haloalkyl or halogen; each of $R^7$ and $R^8$ which are independent of each other, is hydrogen, cyano, alkyl, alkenyl, alkynyl, haloalkyl or cycloalkyl, or $R^7$ and $R^8$ may together form $C_{3-6}$ cycloalkyl which may be substituted by halogen; $R^9$ is alkyl, cycloalkyl, alkoxyalkyl, alkoxyalkoxyalkyl, hydroxyalkyl, halogen, haloalkyl, cyano, nitro, aryl which may be substituted by halogen, a heterocyclic group which may be substituted by halogen, heterocyclic oxy which may be substituted by halogen, $CONR^aR^c$, $COR^a$, $NR^aR^c$, $COOR^a$ or $OR^a$; each of $R^a$ and $R^c$ which are independent of each other, is hydrogen, alkyl, haloalkyl, heterocyclic alkyl, alkoxyalkyl, hydroxyalkyl, cycloalkyl, aryl which may be substituted by $R^d$, or a heterocyclic group which may be substituted by $R^d$; $R^b$ is halogen, aryl which may be substituted by $R^d$, a heterocyclic group which may be substituted by $R^d$, an N-oxide of a nitrogen-containing heterocyclic group which may be substituted by $R^d$, heterocyclic oxy which may be substituted by $R^d$, heterocyclic thio which may be substituted by $R^d$, cyano, $NH^aR^c$, $NHCOOR^a$, $OR^a$, $COR^a$, $COOR^a$ or $S(O)_pR^a$; $R^d$ is alkyl, cycloalkyl, alkoxyalkyl, alkoxyalkoxyalkyl, hydroxyalkyl, halogen, haloalkyl, cyano, nitro, aryl, a heterocyclic group which may be substituted by halogen, heterocyclic oxy which may be substituted by halogen, $COR^a$, amino, monoalkylamino, dialkylamino, $COOR^a$, $OR^a$ or $SR^a$; m is an integer of from 0 to 1; n is an integer of from 0 to 4, p is an integer of from 0 to 2, in the $NR^aR^c$ moiety in each of the above substituents, $R^a$ and $R^c$ may together form a 5- or 6-membered heterocyclic ring together with the nitrogen atom to which they are bonded, provided that 2-[bis(2-pyridinylmethyl)amino]-4-chlorophenol and 2-methoxy-5-chlorophenyl-bis(2-pyridylmethyl)amine are excluded, which comprises reacting a compound represented by the formula (II):

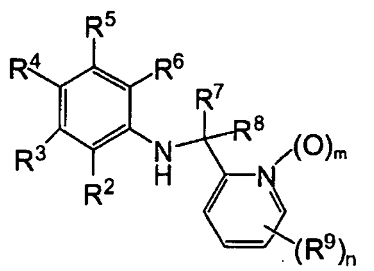

(II)

wherein $R^2$, $R^3$, $R^4$, $R^5$, $R^6$, $R^7$, $R^8$, $R^9$, m and n are as defined above, with a compound represented by the formula (III): $R^1$-X
wherein X is halogen, and $R^1$ is as defined above.

**5.** A pesticide containing, as an active ingredient, the N-phenyl-methanamine derivative or its salt as defined in Claim 1.

**6.** An insecticide, miticide or nematicide containing, as an active ingredient, the N-phenyl-methanamine derivative or its salt as defined in Claim 1.

**7.** An animal parasite-controlling agent containing, as an active ingredient, the N-phenyl-methanamine derivative or its salt as defined in Claim 1.

**8.** A method for controlling a pest, which comprises applying an effective amount of the N-phenyl-methanamine derivative or its salt as defined in Claim 1.

**9.** A method for controlling an animal parasite, which comprises applying an effective amount of the N-phenyl-methanamine derivative or its salt as defined in Claim 1 to the animal parasite.

## INTERNATIONAL SEARCH REPORT

International application No.
PCT/JP2008/061359

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D213/38, A01N43/40, A01N43/60, A01N43/80, A01P7/02, A01P7/04, C07D213/61, C07D213/64, C07D213/70, C07D213/89, C07D401/12, C07D401/14, C07D407/12, C07D409/12, C07D413/12, C07D417/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
REGISTRY(STN), CAplus(STN)

C. DOCUMENTS CONSIDERED TO BE RELEVANT

| Category* | Citation of document, with indication, where appropriate, of the relevant passages | Relevant to claim No. |
|---|---|---|
| X<br>A | US 2004/0224420 A1 (LIPPARD, S.J.), 11 November, 2004 (11.11.04), Par. Nos. [0389], [0391], [0399], [0401]; Fig. 1A (Family: none) | 1-2,4<br>3,5-9 |
| A | YAMAHARA, R. et al., (Catecholato)iron(III) complexes with tetradentate tripodal ligands containing substituted phenol and pyridine units as structural and functional model complexes for the catechol-bound intermediate of intradiol-cleaving catechol dioxygenases, Inorganica Chimica Acta, 2000, Volumes 300-302, pages 587 to 596, particularly, page 588, Scheme 3. compound (a), page 594, right column, 3.2.3 | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 July, 2008 (24.07.08) | 12 August, 2008 (12.08.08) |
| Name and mailing address of the ISA/<br>Japanese Patent Office<br>Facsimile No. | Authorized officer<br>Telephone No. |

Form PCT/ISA/210 (second sheet) (April 2007)

**INTERNATIONAL SEARCH REPORT**

International application No.
PCT/JP2008/061359

C (Continuation). DOCUMENTS CONSIDERED TO BE RELEVANT

| Category* | Citation of document, with indication, where appropriate, of the relevant passages | Relevant to claim No. |
|---|---|---|
| A | JP 2000-226372 A (Nippon Soda Co., Ltd.), 15 August, 2000 (15.08.00), (Family: none) | 1-9 |
| A | JP 2-191253 A (Nippon Tokushu Noyaku Kabushiki Kaisha), 27 July, 1990 (27.07.90), (Family: none) | 1-9 |
| A | JP 3-193763 A (SDS Biotech Kabushiki Kaisha), 23 August, 1991 (23.08.91), (Family: none) | 1-9 |
| A | JP 60-13760 A (Eli Lilly and Co.), 24 January, 1985 (24.01.85), & US 4552960 A & GB 2141714 A & EP 129433 A2 & BR 8402996 A & CA 1240998 A & DK 297084 A & HU 34102 A & IL 72094 A & GR 82366 A & KR 10-1990-0008123 B1 & DK 297084 A0 | 1-9 |

Form PCT/ISA/210 (continuation of second sheet) (April 2007)

**INTERNATIONAL SEARCH REPORT**

International application No.
PCT/JP2008/061359

Continuation of A. CLASSIFICATION OF SUBJECT MATTER
(International Patent Classification (IPC))

*C07D213/38*(2006.01)i, *A01N43/40*(2006.01)i, *A01N43/60*(2006.01)i, *A01N43/80*(2006.01)i, *A01P7/02*(2006.01)i, *A01P7/04*(2006.01)i, *C07D213/61*(2006.01)i, *C07D213/64*(2006.01)i, *C07D213/70*(2006.01)i, *C07D213/89*(2006.01)i, *C07D401/12*(2006.01)i, *C07D401/14*(2006.01)i, *C07D407/12*(2006.01)i, *C07D409/12*(2006.01)i, *C07D413/12*(2006.01)i, *C07D417/12*(2006.01)i

(According to International Patent Classification (IPC) or to both national classification and IPC)

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040224420 A
- US 20030008405 A
- WO 2006113552 A
- WO 2003050174 A
- JP 2007165618 A


### Non-patent literature cited in the description


- *Inorganica Chimica Acta,* 2000, vol. 300-302, 587-596